(19) Europäisches Patentamt
European Patent Office
Office européen des brevets

(11) **EP 2 958 437 B1**

(12) **EUROPEAN PATENT SPECIFICATION**

(45) Date of publication and mention
of the grant of the patent:
**05.02.2020 Bulletin 2020/06**

(21) Application number: **13711367.6**

(22) Date of filing: **21.03.2013**

(51) Int Cl.:
*A23K 10/38* *(2016.01)*     *A23K 10/14* *(2016.01)*
*A23K 20/163* *(2016.01)*    *C12N 9/14* *(2006.01)*
*C12F 3/10* *(2006.01)*      *C12P 7/06* *(2006.01)*
*C12N 9/24* *(2006.01)*

(86) International application number:
**PCT/EP2013/055918**

(87) International publication number:
**WO 2014/127852 (28.08.2014 Gazette 2014/35)**

(54) **PREBIOTIC ANIMAL FEED PRODUCT**

PRÄBIOTISCHES TIERFUTTERPRODUKT

PRODUIT PRÉBIOTIQUE DESTINÉ À L'ALIMENTATION ANIMALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.02.2013 EP 13156260**

(43) Date of publication of application:
**30.12.2015 Bulletin 2015/53**

(73) Proprietors:
• **Direvo Industrial Biotechnology GmbH**
  **50829 Köln (DE)**
• **BASF Enzymes, LLC**
  **San Diego, CA 92121 (US)**

(72) Inventors:
• **ELEND, Christian**
  **50829 Köln (DE)**
• **KRÄMER, Marco**
  **50829 Köln (DE)**

(74) Representative: **Roth, Andy Stefan**
**Dr. Roth Patentanwaltskanzlei**
**Kaistrasse 5**
**40221 Düsseldorf (DE)**

(56) References cited:
WO-A1-2012/084225     WO-A1-2012/166290
US-A- 5 512 287       US-A1- 2003 232 109
US-A1- 2006 233 864   US-A1- 2008 257 821
US-A1- 2009 221 806   US-B1- 6 323 338

EP 2 958 437 B1

## Description

### FIELD OF THE INVENTION

The present disclosure relates to a process of producing a prebiotic animal feed product. The present disclosure relates also to the use of enzymes for improving the prebiotic quality of by-products in a fermentative production process and to compositions comprising enzymes capable of degrading components in the fermented mash in the fermentation process to improve the prebiotic quality of the by-products.

### BACKGROUND OF THE INVENTION

A prebiotic is a non-digestible food ingredient that beneficially affects the host by selectively stimulating the growth and/or activity of one of a limited number of bacteria in the colon, and thus improves health. In general, mammalians can take advantage of prebiotics.

Prebiotics mostly are short chain carbohydrates that alter the composition, or metabolism, of the gut microflora in a beneficial manner. The short chain carbohydrates are also referred to as oligosaccharides, and usually contain between 3 and 10 sugar moieties or simple sugars. Another example for prebiotics are polysaccharides, long carbohydrate molecules of repeated monomer units joined together by glycosidic bonds. When oligosaccharides or polysaccharides are consumed, the undigested portion serves as food for the intestinal microflora. Depending on the type of oligosaccharide, different bacterial groups are stimulated or suppressed.

Oligosaccharides prepared for use in the food and feed industry are not single components, but are mixtures containing oligosaccharides with different degrees of oligomerization, sometimes including the parent disaccharide and the monomeric sugars (Prapulla et al. (2000) Adv Appl microbial 47, 299-343). Various types of oligosaccharides are found as natural components in many common foods, including fruits, vegetables, milk, and honey. Examples of oligosaccharides are galacto-oligosaccharides, lactulose, lactosucrose, fructooligosaccharides, palatinose or isomaltose oligosaccharides, glycosyl sucrose, maltooligosaccharides, isomaltooligosaccharides, cyclodextrins, gentiooligosaccharides, soybean oligosaccharides and xylooligosaccharides (Prapulla et al. (2000) Adv Appl microbial 47, 299-343).

Candidate oligosaccharides, of which the prebiotic potential has been investigated limitedly, are derived from germinated barley, dextrans, pectins, polygalacturonan, rhamnogalacturonan, mannan, hemicellulose, arabinogalactan, arabinan, arabinoxylan, resistant starch, melibiose, chitosan, agarose, alginate (Van Loo, 2005, Food Science and Technology Bulletin: Functional Foods 2: 83-100; Van Laere et al., 2000, J Agric Food Chem 48, 1644-1652; Lee et al. 2002, Anaerobe 8, 319-324; Hu et al, 2006, Anaerobe 12, 260-266; Wang et al, 2006, Nutrition Research 26, 597-603). All of these oligosaccharides are produced using enzymatic processes involving either the hydrolysis of polysaccharides or the synthesis starting from smaller carbohydrates using transglycosylation reactions. In some cases hydrothermal treatment or autohydrolysis is applied to depolymerise lignocellulosic materials, such as xylans (Vazquez et al, 2006, Industrial Crops and Products, 24, 152-159).

Even though polysaccharides are not very digestible, they may also comprise important dietary and health supporting elements for vertebrates and especially for mammalians

For example, beta-glucans are useful as soluble dietary fibers (reviewed by Petravic-Tominac et al., 2010. Conspectus Scientificus 75 (4): 149-158) Soluble fibre remains undigested except by colonic microflora in the lower intestines. This enhances the growth of bacteria beneficial to health. Soluble dietary fibre is believed to have a role in the prevention of certain diseases including colonic cancer and diseases associated with high serum cholesterol levels. Soluble fibre can be used for the treatment and prevention of constipation, for the improvement of bowel regularity, and for the regulation of the glycaemic response associated with the digestion of many substances.

Beta-glucans are considered to have hypocholesterolemic activity. beta-glucans are also useful as food ingredients. They have neutral flavors and provide bulk in addition to having desirable mouthfeel and texture characteristics. In this context, beta-glucans are known as fat replacements in some foods.

Beta-glucan activates also the immune response through the immune cells, called macrophages, showing various therapeutic effects The immune system is primary natural defense against any disease and even aging. But, beta-glucans are not synthesized by human body so they have to be recognized by immune systems, inducing immune responses (Brown and Gordon, 2005. Cell Microbiol 7:471-479). In vitro and in vivo studies in animals and humans show that beta-glucans derived from fungi and yeasts have immunomodulating properties. Most frequently evaluated are their effects on leukocyte activity. Immune response is influenced by both parenteral and enteral administration of beta-glucan (Volman et al., 2008. Physiology and Behavior 94: 276-284). Beta-glucan nutritionally promotes regeneration of immune system. The immunoregulatory activities of beta-glucan relate mainly to its ability to stimulate or inhibit macrophage release of cytokines involved in immune system control or to modulate macrophage phagocytosis (Bohn and BeMiller, 1995. Carbohyd Polym 28: 3-14; Gardiner, 2000. Glycoscience and Nutrition 1: 1- 6; Vetvicka et al., 2002. JANA 5: 1-6; Vetvicka et al., 2007. Int J Biol Macromol 40: 291-298). In *vivo study* in animals has shown that orally administered beta-glucan activates white blood cells, such as macrophages, granulocytes and monocytes, responsible for defense against infections, and supports the repair of damaged tissues in the body (Vetvicka et al., 2002. JANA 5: 1-6; Rice et al., 2002. J Leukocyte Biol 72: 140-146). In this regard yeast beta-glucan with (1→3) (1→6)-glycosidic linkage has demonstrated particularly strong immunomodulatory activity influencing the immune response of the host and is oft en described as biological response modifyer (DiLuzio, 1983. Trends Pharmacol Sci 4: 344-347). All cells involved in immune reactions originate from common precursors - stem cells originating from bone marrow (Kougias et al., 2001. Infect Immun 69: 3933-3938; Rice et al., 2002. J Leukocyte Biol 72: 140-146). So, beta-glucan stimulates the production of precursor cells in bone marrow, resulting in a bloodstream flow of new immunocytes into the various lymphoid organs throughout the body. The increased amount of immunocytes in circulation means increased protection from potential invaders. It is important particularly in case of extreme stress (e.g. in case of cancer), when immune system is exhausted by treatments such as irradiation and chemotherapy (Kougias et al., 2001. Infect Immun 69: 3933-3938; Hong et al., 2003. Cancer Res 63: 9023-9031). Beta-glucan did not show any genotoxic and/or clastogenic damage, so it may become an important adjuvant in chemotherapy while it has capacity to diminish adverse effects of drugs (Oliveira et al., 2006. Toxicol In Vitro 21: 41-52).

Another important biological activity of beta-glucan is prevention of bacterial infection. It can elevate the general level of resistance to pathogens and reduce the risk of infections. Numerous studies and clinical trials have proved that soluble beta-glucans improve resistance to bacterial infection (Kernodl et al., 1998. Antimicrob Agents Chemother 42: 545-549; Dellinger et al., 1999. Arch Surg 134: 977-983). Orally-administered insoluble yeast beta-glucan (2-20 mg/kg) provided a maximal antrax-protective effect in mice, without using antibiotics (Vetvicka et al., 2002. JANA 5: 1-6). β-(1→3)(1→6)-glucan increases resistance to many diseases enhancing leukocyte anti-infective activity in human whole blood, without increasing the inflammatory cytokine production (Wakshull et al., 1999. Immunopharmacology 41: 89-107). *Saccharomyces cerevisiae* beta-glucan extract was shown to have antimicrobial activity in mice, against *Staphylococcus aureus* resistant to antibiotics. Beta-glucan administration helps in the elimination of bacteria and increases the number of monocytes and neutrophils. *In vitro* experiments on rats improved the full action of beta-glucans and antibiotics against *Escherichia coli* and *Staphylococcus aureus* (Tzianabos and Cisneros, 1996. Ann N.Y. Acad Sci 797: 285-287). It is an example of synergism of beta-glucan and antibiotics. Special attention has been paid to post-surgical infection, which occurs in 25-27% of cases. For that reason, practical human clinical trials of beta-glucan impact for reducing the incidence of infection were studied (Dellinger et al., 1999. Arch Surg 134: 977-983). The results of clinical trials demonstrated that beta-glucan therapy reduce postoperative infections by 39%. Studying the addition of injectable form of beta-glucan in humans, most researchers have concluded that yeast beta-glucan promotes phagocytoses and subsequent killing of phathogenic bacteria (Tzianabos and Cisneros, 1996. Ann N.Y. Acad Sci 797: 285-287; Kernodle et al., 1998. Antimicrob Agents Chemother 42: 545-549; Dellinger et al., 1999. Arch Surg 134: 977-983). The *in vivo* study on rats showed that systemic beta-glucan treatment would result in enhanced migration of neutrophils into a site of inflammation and improve antimicrobial function (LeBlanc et al., 2006. J Leukoc Biol 79: 667-675). The similar trials have been done with the orally consumed beta-glucan. The studies proved that oral consumption of beta-glucan pills are as effective as the injections in improvement of natural immunity (Vetvicka et al., 2007. Int J Biol Macromol 40: 291-298). Some researchers investigated the ability of yeast beta-glucan to reduce septic infection using *in vivo* models. Onderdonk et al. (1992. Infect Immun 60: 1642-1647), in their early studies, found that mice provoked with *Escherichia coli* or *Staphylococcus aureus* bacteria were protected against septic infection if they were injected by PGG-glucan 4-6 hours before the incubation. On the other hand, Kernodle et al. (1998. Antimicrob Agents Chemother 42: 545-549) showed that preventive amounts of yeast beta-glucan prior to infection with *S. aureus* inhibited the sepsis in a guinea pig. Additional research work supports the theses that yeast beta-glucan reduces septic shock by killing bacteria in blood (DiLuzio et al., 1979. Int J Cancer 24: 773-779; Kulicke et al., 1997. Carbohyd Res 297: 135-139; Liang et al., 1998 ; Int J Immunopharmacol 20: 595-614 Tzianabos et al., 1998. J Infect Dis 178: 200-206; Chen and Seviour, 2007. Mycol Res 111: 635-652).

Despite of immunomodulating and antibacterial effects of beta-glucans lowing of cholesterol and wound healing was found. Beta-glucans from various sources have effective cholesterol and lipids lowering properties (Mason, 2001. What is beta-glucan? In:Safe goods. New Century Publishing 2000, pp 4-36. Printed in USA: http://www. youngagain.org/book_what_is_beta_glucan.htm). Worldwide human studies have demonstrated that beta-glucan is a safe, powerful and inexpensive way to lower blood cholesterol and high lipid level (Nicolosi et al., 1999. Am J Clin Nutr 70: 208-212; Bell et al., 2001. US Patent 6,210,686; Keogh et al., 2003. Am J Clin Nutr 78: 711-718; Steriti, 2007. Dynamic Chiropractis 7: 1-6). Lowering the blood lipids and total and LDL cholesterol is the best way to prevent high blood pressure and arteriosclerosis. Natural preparations of beta-glucans like oat milk, barley or yeast beta-glucans preparations added to juicy drinks could be used instead of expensive drugs with dangerous side-effects (Naumann et al., 2006. Amer J Clin Nutrit 83: 601-605). It is also known that macrophage activity plays a main role in wound healing, from surgery or trauma. There have been numerous clinical studies with soluble yeast beta-glucan and the whole glucan particles. In both animal and human studies, therapy with beta-glucan showed improvements like reduced mortality, lowered infection, and stronger tensile strength of scar tissue (Browder et al., 1990. Ann Surg 211: 605-613; Portera et al., 1997. J Leukocyte Biol 72: 140-146). Based on these results they have concluded that immunomodulators that enhance macrophage activity have positive effects on collagen biosynthesis in the healing wounds in experiments using animal and human models.

It is also shown that beta-glucan is active in a broad spectrum of biological species that is important for its application (Vetvicka, 2001. JANA 3: 31-34). Consequently, it could be applied in production of feed and veterinary drugs, used for pets and economically important animals. Application of beta-glucan in animal farm feed lowered antibiotics consumption and reduced harmful residues in food (Hayen and Pollmann, 1995, international patent application WO 95/04467). By the use of beta-glucan as feed additive during intensive growth of poultry, cattle, fish and crabs, infections were lowered, improving growth and lowering needs for antibiotics addition (Donzis, 1993. US Patent 5,223,491). Investigations of substances acting non-specifically on immunoreactions are becoming more and more significant from the point of view of veterinary medicine. Commercially important animals are grown in stressful conditions and it is important to stimulate their immunity by immunostimulants to lower mortality induced by stress. In animals, beta-glucan was tested not only as specific stimulant but also as addition to currently used vaccines (Vetvicka, 2001. JANA 3: 31-34). Beta-Glucan was at first used to prevent infections in hatchery raised fish (Keller, 2000. International Journal of Pharmaceutical Compounding 4: 342-345). Nikl and Allbright (1993. US Patent 5,189,028) explained the possibility of stimulating the immune system of a fish *Salmonidae* and enhancing the efficacy of vaccines for treating fish diseases. The application of beta-glucan in combination with vitamins improves the resistance of aquatic animals and especially of fish, shrimps and invertebrates, as well as warm and cold water decorative fish (Kürzinger, 2001. US Patent 6,306,453 B1). Cook et al. (2003. Fish Shellfish Immun 14: 333-345) investigated the effects of prolonged administration of a commercial beta-glucan based immunostimulant preparation, in the form of a feed supplement, on non-specific immune parameters and the growth rate of snapper (*Pagrus auratus*). The preparation contained glucan and mannan and the average particle dimensions were less than 1 $\mu$m. Their results suggest that routine incorporation of similar beta-glucan preparations in fish feed has beneficial effect. Hayen and Pollmann (1995. WHO Patent 95/04467) investigated the orally administering the animal with glucan isolated from the yeast, as the component of feed for enhancing animal growth. Preparations containing glucan and mannan, isolated from spent brewer's yeast, which was added into a feed in concentration of 0.1% w/w showed its efficiency as immunoactivators and diminished the mortality at fishes and crabs due to infection (0.1% w/w in the food). The same product when added in concentration of 1 g/kg into poultry feed resulted in high resistance to diseases (Lee et al., 2003. Biomaterials 24: 2503-2511).

The reported methods of extracting beta-glucans from cereal involve a number of steps. First, the milled cereal is treated to deactivate enzymes associated with the cereal. Then the beta-glucan is extracted from the cereal into warm water after which the solids are removed from the solution.

Large quantities of water-miscible organic solvents are added to the solution to precipitate the (beta-glucan, generally along with other polysaccharides. The beta-glucan is of low purity and of generally high molecular weight.

It is also known to carry out the enzyme deactivation step after the water extraction rather than as a first step. The deactivation step inhibits hydrolysis of the beta-glucan thereby maintaining a high average molecular weight of the beta-glucan. US-A-5512287 describes a method for obtaining beta-glucan from cereal grains, which involves the step of destroying beta-glucanase activity.Mannose is a monosaccharide that forms the building block of mannan-oligosaccharides (mos) (reviewed by Spearman, 2004). The small intestine does not contain the digestive enzymes required to break down mannan oligosaccharide bonds, therefore they arrive at the large intestine intact after ingestion and passage through the small intestine (Strickling et al. 2000. An Feed Sci & Tech 86: 205-219). Mannan-oligo-saccharides occur naturally in cell walls of the yeast *Saccharomyces cerevisiae* and can relatively easy to obtain by centrifugation from a lysed yeast culture (Spring et al. 2000. Poult Sci 79: 205-211). The commercially available products like Bio-Mos® (Alltech, Inc., Nicholasville, KY) is a source of mos from *Saccharomyces cerevisiae* cell walls. This product was introduced in 1993 as a feed additive for broiler chickens (Hooge 2003. Feedstuffs 75(1)). Lectins are carbohydrate-binding proteins that mediate interactions of cells with their environment through their initial interactions with other cell surface carbohydrates (Osborn and Khan 2000. Oligosaccharides: Their synthesis and biological roles. Oxford University Press Inc., New York). Mannose residues on the surface of intestinal epithelial cells serve as receptor binding sites for certain pathogens with type-1 fimbriae that contain mannose-specific lectins (Ofek and Beachey 1978. Infection and Immunity 22(1): 247-253, Oyofo et al. 1989b Poult Sci 68: 1351-1356, Spring et al. 2000. Poult Sci 79: 205-211, Röckendorf et al. 2002. Aust J Chem 55: 87-93). Adherence to the intestinal cell wall is a prerequisite for the initiation of colonization by pathogenic organisms in the gastrointestinal tract (Ferket et al. 2002. Benefits of Dietary Antibiotic and Mannanoligosaccharide Supplementation for Poultry. Proc Multi-State Poultry Meeting. Indianapolis, Indiana, May 14-16, 2002). Once binding by the pathogenic organism occurs, translocation across the intestinal wall and subsequent enteric infection can occur (Iji et al. 2001. J Sci Food Agric 81: 1186-1192, Ferket et al. 2002. Benefits of Dietary Antibiotic and Mannanoligosaccharide Supplementation for Poultry. Proc Multi-State Poultry Meeting. Indianapolis, Indiana, May 14-16, 2002).

Mannan oligosaccharide preparations have been shown to agglutinate pathogens with mannose-specific type-1 fimbriae in vitro. Spring et al. (2000. Poult Sci 79: 205-211), in an attempt to investigate the ability of different enteric pathogens and coliforms to trigger mos agglutination, showed that mos agglutinated 7 of 10 strains *Salmonella. typh-*

*imurium* and *Salmonella. enteritidis* and 5 of 7 strains of *E. coli* in vitro. Intestinal Environment Studies Efforts to demonstrate that mos has the same effect on bacterial populations in the intestinal environment have proven successful. Oyofo et al. (1989b. Poult Sci 68: 1351-1356) investigated the adherence of *Salmonella typhimurium* to the small intestine of one-day-old chicks and found that adherence was significantly inhibited in the presence of D-mannose. Droleskey et al. (1994. Avian Diseases 38: 275-281) found that incubation of *Salmonella typhimurium* with cultured chick intestinal segments resulted in the loss of mucosal epithelial integrity evidenced by the complete shedding of the epithelium. It was found in this study that the addition of 2.5% D-mannose to the incubation medium inhibited the loss of epithelial cells. When provided in the drinking water of chicks, mannose significantly reduced intestinal colonization of *S. typhimurium* (Oyofo et al. 1989a. Avian Diseases 33: 531-534). When supplemented to the diet of hens, mos affected the birds' intestinal microflora by increasing the *Bifidobacterium* spp. and *Lactobacillus* spp., while decreasing colonization of *Salmonella enteritidis* (Fernandez et al. 2002. Avian Pathology 31(1), 49-58). The addition of 4,000 ppm of mos to the diet of threeday- old chicks that were orally challenged with *Salmonella typhimurium* significantly reduced cecal *Salmonella typhimurium* concentrations on day 10 when compared with controls (Spring et al. 2000. Poult Sci 79: 205-211). In a separate trial using *Salmonella dublin* as the challenge organism, the number of chicks that tested positive for Salmonella in the cecum at day 10 was less in chicks that were consuming the mos supplemented diet (Spring et al. 2000. Poult Sci 79: 205-211). In growing turkeys younger than six weeks of age, mos supplemented birds had a higher total anaerobe count and a lower level of *Clostridium perfringens* in cecal cultures (Finuance et al. 1999. Poult Sci 78(Suppl 1):77).

These studies demonstrate that pathogens with the mannose-specific type-1 fimbriae adsorb to mos instead of attaching to intestinal epithelial cell walls and, therefore, move through the intestine with less probability of initiating disease. There have also been investigations into the intestinal environment effects of mos supplementation to the diet of a companion animal species. Strickling et al. (2000. An Feed Sci & Tech 86: 205-219) found that in dogs, fecal *Clostridium perfringens* tended to be lower when supplemented with 5g mos/kg diet DM. The same study found no diet effects on fecal bifidobacteria numbers or ileal bacteria colony forming units. Dogs supplemented with 2 g mos/day had significantly lower fecal total aerobe and tended to have greater *Lactobacillus* populations (Swanson et al. 2002. J Nutr 132: 980-989). 1 g/kg BW/day of mos supplementation to the diets of 4 female beagle dogs resulted in a lower fecal pH (Zentek et al. 2002. J Nutr 132: 1682S-1684S).

The use of antibiotics in food animal diets is a common practice in the industry. Antibiotics have been shown to improve growth, feed efficiency, and overall herd health when used in poultry, swine, and cattle production diets. Due to consumer concerns and increasing regulatory restrictions, producers have begun searching for alternatives to the use of antibiotic growth promotants in production diets. Mannan oligosaccharide supplementation has been investigated as an alternative to antibiotic supplementation to enhance performance characteristics (Spearman, 2004. Effect on Mannan oligosaccharide (mos) supplementation on the immune status of mares and their foals. Master thesis at the university of Florida).

Numerous studies have been conducted in poultry (reviewed by Spearman, 2004. Effect on Mannan oligosaccharide (mos) supplementation on the immune status of mares and their foals, because mos was first introduced in 1993 as a feed additive for broiler chicken diets (Hooge 2003. Feedstuffs 75(1)). Over 150 broiler chicken pen trials were analyzed to collectively determine the effects of mos-supplemented diets versus negative and/or positive control (antibiotic) diets. The conclusion was that mos supplementation results in bodyweight and feed conversion ratios comparable to antibiotic supplementation while significantly lowering mortality rate (Hooge 2003. Feedstuffs 75(1)). Fritts and Waldroup (2000. Poult Sci 79(Suppl 1): 126) reported that turkey poults fed 0.10% mos had the same feed conversion as poults fed 55 ppm of the antibiotic bacitracin methylene disalicyclate (BMD) and significantly better feed conversion than negative controls. In a study conducted to determine growth effects in turkey hens with diets supplemented with mos or antibiotics (BMD and virginiamycin), investigators found that birds fed 0.5g/ kg mos supplemented birds had improved feed efficiency over birds fed the control or antibiotic-supplemented diet (Hulet et al. 2000. Poult Sci 79 (Suppl 1): S186). Mannan oligosaccharide was shown to be a suitable alternative to terramycin as a growth enhancer in turkey diets when no difference in bodyweight was seen between control and treatment animals after 105 days of supplementation (Stanley et al. 2000. Poult Sci 79 (Suppl 1): S186). Both mos and antibiotic growth promoters enhance the efficiency of nutrient utilization by reducing the competition between the host and intestinal pathogens. Without microbial competition for energy and other nutrients, there are more nutrients available for absorption and metabolism by the host (Ferket et al. 2002. Benefits of Dietary Antibiotic and Mannanoligosaccharide Supplementation for Poultry. Proc Multi-State Poultry Meeting. Indianapolis, Indiana, May 14-16, 2002). It is well documented that antibiotic supplementation to poultry diets increases the utilization of dietary energy (Buresh et al. 1985. Poult Sci 64: 1041-1042, Harms et al. 1986 Poult Sci 65: 1984-1986, Ferket et al. 2002. Benefits of Dietary Antibiotic and Mannanoligosaccharide Supplementation for Poultry. Proc Multi-State Poultry Meeting. Indianapolis, Indiana, May 14-16, 2002). Although mos supplementation has proved to be as effective as antibiotics in improving utilization of dietary energy, the mechanism is unclear and likely different than that used by antibiotic growth promotants. Possibly it is related to the improvement of characteristics of the intestinal lining (Ferket et al. 2002. Benefits of Dietary Antibiotic and Mannanoligosaccharide Supplementation for Poultry. Proc Multi-State Poultry Meeting. Indianapolis, Indiana, May 14-16, 2002) or changes in digestive enzyme activities that are stimulated by mos (Iji et al. 2001. J Sci Food Agric 81: 1186-1192).

Pregnant sows fed 0.20% mos three weeks prior to farrowing and 0.10% mos throughout the 21-day lactation period produced piglets with heavier litter birth and weaning weights (O'Quinn et al. 2001. J Anim Sci 79: 212). In a factorial experiment conducted to determine the effects of two levels of mos (0 and 0.10%) and three levels of protein (20, 23, and 26%) in piglet diets, mos supplementation improved weight gain and feed consumption regardless of protein level (Kim et al. 2000. J Anim Sci Tech 42(4): 489-498.). The addition of minerals such as Zn and Cu in excess of NRC recommendations to swine diets is a common practice to improve performance (NRC 1998. National Research Council. Nutrient Requirements of Swine. 10th ed. National Academy Press, Washington, DC). However, this may result in an undesirable effect on the bacteria responsible for waste degradation in lagoons (Gilley et al. 2000. Trans Am Soc Agric Eng 43: 1853-1859). The addition of 0.20% mos to the diets of nursery pigs increased average daily gain and average daily feed intake in the absence of excess zinc but had no effect or a negative effect in the presence of excess zinc (LeMieux et al. 2003. J Anim Sci 81: 2482-2487). In a separate trial of the same study, the interactive effects of antibiotics (oxytetracycline and neomycin) and mos and of Zn and mos were evaluated. Mannan oligosaccharide improved pig performance only when fed in combination with an antibiotic and no excess Zn. There was no effect or a negative effect in the presence of excess Zn or in the absence of an antibiotic (LeMieux et al. 2003. J Anim Sci 81: 2482-2487). Mannan oligosaccharides have also been considered as an alternative to excess Cu supplementation in swine diets for performance enhancement. The effects of mos fed at either basal or excess levels of Cu in the diets of weanling and growing-finishing pigs were determined in an experiment by Davis et al. (2002. J Anim Sci 80: 2887-2894). From day 0 to day 10, average daily gain, average daily feed intake, and gain: feed increased when mos was added to diets containing basal levels of Cu. From day 10 to day 38, pigs fed diets containing excess Cu had greater ADG and ADFI regardless of mos addition (Davis et al. 2002. J Anim Sci 80: 2887-2894).

The production-enhancement effects of mos supplementation in cattle diets have received relatively less attention than supplementation of poultry or swine diets. Heinrichs et al. (2003. J Dairy Sci 86(12): 4064-4069) investigated the effects of mos or antibiotics in dairy calf milk replacer diets, and found the addition of 4 g mos/day was as effective as antibiotic use to maintain normal fecal fluidity and consistency and to decrease scours severity. Feed consumption increased when mos was included in the diet, but this did not result in a difference in growth measures (Heinrichs et al. 2003. J Dairy Sci 86(12): 4064-4069).

After mos supplementation to production diets proved to increase weight gain and feed efficiency, identifying the mechanism of the physiological response associated with the positive growth responses was the next logical step. To do this, studies focused on measuring the parameters that are representative of a functional immune system. These parameters include Ig content of the serum, lymphocyte proliferation, and response to antigenic stimulation. The main antigenic components of yeast cells are mannans present in the isolated cell wall (Ballou 1970. J Biol Chem 245(5): 1197-1203). Mannans found in the cell walls of *Saccharomyces cerevisiae* have been shown to induce an antigenic response in humans (Young et at. 1998. Glycoc J 15: 815-822) Therefore some mos-immune system interaction would be expected (Ferket et al. 2002. Benefits of Dietary Antibiotic and Mannanoligosaccharide Supplementation for Poultry. Proc Multi-State Poultry Meeting. Indianapolis, Indiana, May 14-16, 2002). Poultry. Savage et al. (1996. Poult Sci 75 (Suppl 1)) fed 0.11% mos to male turkeys for 53 days and obtained blood and bile samples at the end of the period. The samples were analyzed using both radial immunodiffusion (RID) and rocket immunoelectophoresis (RI). Using RID, no significant differences were found, but RI analysis showed that concentrations of both blood and bile IgG and IgA were significantly increased in turkeys fed mos (Savage et al. 1996. Poult Sci 75 (Suppl 1)). In a trial investigating the effects on humoral immunity in commercial laying hens, investigators injected the hens with sheep red blood cells (SRBC) suspended in a solution of bovine serum albumin (BSA) and obtained serum samples one, two, and four weeks post-sensitization. Hens supplemented with 0.05% mos had higher SRBC titers than controls at one week post-sensitization (Malzone et al. 2000. Poult Sci 79(Suppl 1): 165). The BSA titers of the mos-fed hens were numerically greater at week one and week two, but the differences were not statistically significant (Malzone et al. 2000. Poult Sci 79(Suppl 1): 165). In broiler breeder diets, the addition of mos significantly increased the antibody response to infectious bursal disease virus and also increased maternal antibody titers in the breeders' progeny (Shashidhara and Devegowda 2003. Poult Sci 82(8): 1319-1325).

Positive immune response effects have also been observed with mos supplementation to swine diets. Newman and Newman (2001. J Anim Sci 79: 189) supplemented sow diets with 5g mos/ day for approximately 14 days prefarrowing and continued supplementation throughout lactation. At farrowing, mos treated sows had significantly higher serum IgM and colostrum IgM levels and numerically higher colostrum IgG levels (Newman and Newman 2001. J Anim Sci 79: 189). The piglets from the mos treated sows also weighed more on day 7, 14, and 21 postfarrowing than those from unsupplemented sows (Newman and Newman 2001. J Anim Sci 79: 189). In another study evaluating sow and litter performance, concentrations of IgA, IgG, and IgM in pre-suckle colostrum samples were increased by mos addition to the diet. IgG showed the greatest response, followed by IgM and IgA respectively (O'Quinn et al. 2001. J Anim Sci 79: 212). As found in the previous trial, the piglets from the mos treated sows also had heavier litter birth and weaning weights (O'Quinn et al. 2001. J Anim Sci 79: 212). To determine whether mos modulated the cell-mediated immune response of the weaned pig, Davis et al. (2002. J Anim Sci 80: 2887-2894) obtained blood samples from mos supplemented growing-finishing pigs and measured lymphocyte proliferation in vitro. Lymphocyte proliferation did not differ significantly between the control and MOS supplemented pigs (Davis et al. 2002. J Anim Sci 80: 2887-2894). Although not demonstrated in this study, other researchers have demonstrated that mos may have an inhibitory effect on certain lymphocyte functions (Muchmore et al. 1990. J Leuko Biol 48: 457-464, Podzorski et al. 1990. J Immunol 144: 707-716). It is conceivable that immune function suppression could also be a means by which mos improves gain and efficiency, because of the shift in metabolic activity to support the body's defense against foreign antigens that occurs during immune response activation (Spurlock 1997. J Anim Sci 75: 1773-1783). Another mechanism of growth enhancement in swine may be through the alteration of intestinal microflora, as is what happens with supplementation with pharmacological levels of Cu (Davis et al. 2002. J Anim Sci 80: 2887-2894). Cattle. The addition of 10 g mos/ day to the diet of 40 dairy cows resulted in numerically greater serum Ig levels in calves 24-hours post-calving than in the calves of unsupplemented cows (Franklin et al. 2002. J Anim Sci 80 (Suppl): 192). In the same study, antibody titers to rotavirus vaccination following calving were numerically greater in claves from mos supplemented cows (Franklin et al. 2002. J Anim Sci 80 (Suppl): 192).

Adult female dogs were supplemented with 1 g mos per day for a 14 day period, and serum IgA concentrations tended to be greater and the percent of white blood cells that were lymphocytes was greater in dogs supplemented with mos. Total white blood cell count and neutrophil concentration were unaffected by treatment (Swanson et al. 2002. J Nutr 132: 980-989). The authors hypothesized that because serum IgG and IgM were not affected, a systemic immune response may not have occurred and was not the cause of the increased lymphocytes and serum IgA. The trends for increased serum IgA and lymphocyte concentration may be due to the increased proliferation of Blymphocytes and secretory IgA in the intestinal tract (Swanson et al. 2002. J Nutr 132: 980-989). A study performed in rats reported increased cecal IgA contents and an increase in the proportion of IgA-presenting lymphocytes present in the cecal mucosal of rats fed glucomannans at 5% for three weeks (Kudoh et al. 1999. J Nutr Sci Vitaminol (Tokyo) 45(2): 173-181). These studies have demonstrated the positive effects of mos on Ig concentration in serum and colostrum and on immune response to antigen challenge. However, a mechanism for this action has yet to be demonstrated. Some studies suggest that mos supplementation stimulates intestinal lymphoid tissue resulting in increased development or activation (Guigoz et al. 2002. Nutr Res 22, 13-25,). The stimulatory effect may occur through a healthy population of gut microflora or "drag effects" of the indigestible oligosaccharide molecules as they move along the length of the intestine (Cunningham-Rundles and Lin 1998. Nutrition 14, 573-579). The activation of lymphoid tissue may result in greater plasma cell production by B-cells found in underlying lymphoid follicles. These plasma cells then would be able to secrete Igs that can either be secreted into the intestinal lumen when associated with secretory component or end up in the circulation via transport through the lymphatic system. To determine if mos supplementation to the diet of pregnant mares would result in a change in the total Ig concentration of the mare's colostrum and the serum of the mare or foal, the current experiment was proposed. Previous results from work in other species suggest that mos supplementation will increase colostrum Ig content, and therefore translate into increased foal serum Ig content after absorption of maternal antibodies is complete. Growth measurements of both mares and foals will indicate any negative effects of mos supplementation on physical development. Determination of Ig content in serum and presuckle colostrum samples will indicate any change of immune status in the mare. Serum Ig concentration in the foals will reflect any effect on absorption of colostral Igs and initial serum Ig concentration and any long-term effect on immune status of the foal due to mos supplementation of the dam (Spearman, 2004. Effect on Mannan oligosaccharide (mos) supplementation on the immune status of mares and their foals. Master thesis at the university of Florida).

Wo 202/084225 A1 relates to improved processes of producing fermentation products from starch-containing material using a fermenting organism. In particular, the processes relate to the conversion of corn to ethanol.

US 2009/221806 A1 descibes a process for preparing beta-, 1,3-glucans from a glucan-containing matrix. For example, the matrix can be a fermentation broth or a culture medium.

Therefore, it is an object of the present disclosure to provide methods for manufacturing improved animal feed with a high content of prebiotics like beta glucan and mannan-oligo-saccharides (mos).

## SUMMARY OF THE DISCLOSURE

The present disclosure, which does not describe part of the present invention, relates to processes of producing a prebiotic animal feed product comprising a by-product derived from a fermentative production process comprising the steps of: i) subjecting the fermented mash after the fermentation to an enzyme composition capable of degrading one or more fermented mash components, ii) separating the desired primary fermentation product, iii) separating the fermentation by-product having improved prebiotic quality.

However, the present invention is only defined by the claims. The terms "embodiments" and "aspects of the invention" etc. as listed below, are for illustrative purposes alone.

In particular, one aspect of the present disclosure pertains to methods for the improvement of the quality of the by-products or residues derived from fermented mash comprising the steps of subjecting the fermented mash after the fermentation to an enzyme composition comprising an enzyme or a mixture of enzymes capable of degrading one or more fermented mash components to the prebiotica beta glucan (gos) and / or mannan-oligo-saccharides (mos).

In another aspect, the present disclosure relates to methods of producing a prebiotic animal feed product comprising a fermentation by-product having a high content of prebiotics like beta glucans and/or mannan-oligo-saccharides from starch-containing material in fermentation production process, said method comprising the steps of:

i) Converting the starch-containing material to fermentable sugars,
ii) Fermentation of the fermentable sugars with a fermenting microorganism to fermented mash,
iii) Subjecting the fermented mash after the fermentation process to an enzyme composition capable of degrading one or more fermented mash components,
iv) Separation of the primary product in the fermented mash,
v) Separation of the fermentation by-product.

The present disclosure relates also to methods of producing a prebiotic animal feed product comprising a fermentation by-product having a high content of prebiotics like beta glucans and/or mannan-oligo-saccharides from starch-containing material in an alcohol fermentation production process, said method comprising the steps of:

i) Converting the starch-containing material to fermentable sugars,
ii) Fermentation of the fermentable sugars with a fermenting microorganism to fermented mash,
iii) Separation of the alcohol in the fermented mash by distillation,
iv) Subjecting the remaining whole stillage to an enzyme composition capable of degrading one or more components of the whole stillage to beta glucans and/or mannan-oligo-saccharides,
v) Separation of the by-product.

In a further aspect, the present disclosure pertains to the use of an enzyme composition comprising a beta-1,3-glucanase and/or a xylanase for the improvement of the prebiotic quality of a by-product derived from fermented mash in a fermentative ethanol production process.

## BRIEF DESCRIPTION OF THE DRAWING

Figure 1 schematically shows an ethanol production process.

Figure 2 schematically shows an ethanol process including on site fermentation tank for enzyme production based on WDG.

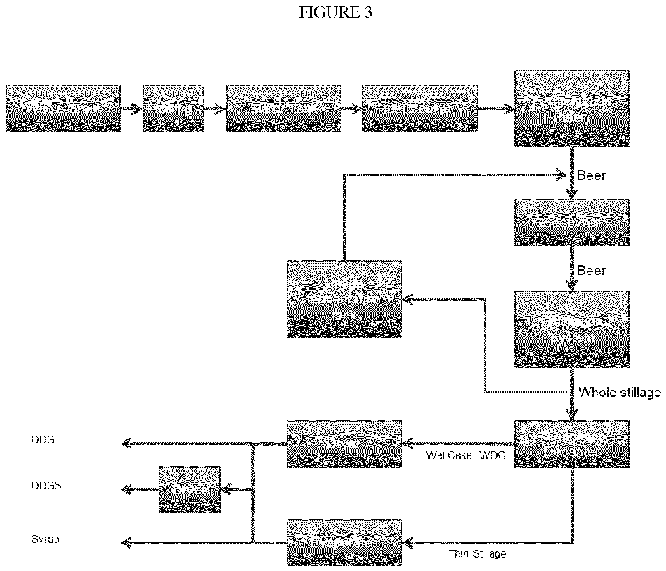

Figure 3 schematically shows an ethanol process including on site fermentation tank for enzyme production based on whole stillage.

Figure 4 is a diagram showing the increase of beta-glucans in DDGS extract by using beta-glucanase.

Figure 5 is a diagram showing the increase of mannan-oligo-saccharides (mos) in DDGS extract by using beta-glucanase.

## DESCRIPTION OF THE INVENTION

The object of the present invention is to provide improved by-products from a fermentation process with an improved quality in respect to content of prebiotics.

Fermentation products, such as ethanol, are produced by first degrading starch- containing material into fermentable sugars by liquefaction and saccharification and then converting the sugars directly or indirectly into the desired fermentation product using a fermenting organism. Liquid fermentation products such as ethanol are recovered from the fermented mash (often referred to as "beer" or "beer mash"), e.g., by distillation, which separate the desired fermentation product from other liquids and/or solids. The remaining faction, referred to as "whole stillage", is dewatered and separated into a solid and a liquid phase, e.g., by centrifugation. The solid phase is referred to as "wet cake" (or "wet grains" or "WDG") and the liquid phase (supernatant) is referred to as "thin stillage". Dewatered wet cake is dried to provide "Distillers Dried Grains" (DDG) used as nutrient in animal feed. Thin stillage is typically evaporated to provide condensate and syrup (or "thick stillage") or may alternatively be recycled directly to the slurry tank as "backset". Condensate may either be forwarded to a methanator before being discharged or may be recycled to the slurry tank. The syrup consisting mainly of limit dextrins and non-fermentable sugars may be blended into DDG or added to the wet cake before drying to produce DDGS (Distillers Dried Grain with Solubles).

It is known to commercially use the various byproducts and residues derived from the fermentation processes like the ethanol production process. Distillers residues or byproducts, as well as by-products of cereal and other food industry manufacturing, are known to have a certain value as sources of protein and energy for animal feed. Furthermore, the oil from the by-products like DDGS can be recovered as a separate by-product for use in biodiesel production or other biorenewable products are sought.

The by-products like DDG, DDGS,WDG or syrup comprises proteins, fibers, fat and unconverted starch. Also yeast cells, the fermenting organism converting glucose to ethanol are present, typically about 6% (g/g) of the total by-products. Cell wall of Saccharomyces and other kinds of yeast represents 26 - 32 % of the yeast dry weight. This cell wall is a structural component and gives the yeast its shape and rigidity. The yeast cell wall is made of 30 - 60 % polysaccharides consisting of beta-glucan and mannan sugar polymers, 15 -30 % proteins, 5 - 20 % lipids and a small amount of chitin. Most of the protein is linked to the mannan-oligo-saccharides (mos) and is referred to as the mannoprotein complex. Typically yeast cell wall contains 15 - 30 % beta-glucan and 15 - 30 % mos.Beta glucan and mannan-oligo-saccharides (mos) have several beneficial properties and therefore show a wide variety of useful applications in human and in veterinary medicine, pharmaceutical, cosmetic and chemical industries as well as food and feed production (Zekovic et al., 2005; Laroche and Michaud, 2007), which are known as prebiotic activity in humans and in animals. Prebiotics are non-digestible food ingredients that promote health in several manners.

One aspect of the present disclosure relates to methods for improving the quality of by-products or residues derived from starch-containing material in a processes for producing fermentation products comprising the steps of: i) subjecting the fermented mash after the fermentation to an enzyme composition comprising an enzyme or a mixture of enzymes capable of degrading one or more fermented mash components, ii) separating the desired fermentation product.

By-products or residues of the fermenting process includes distillers' grain, brewer's grains, dried distiller's grains, dried distiller's solubles, distiller's dried grains with solubles, WDG or/and residues of the cereal processing industry, or mixtures thereof. For example, DDGS are the dried residue remaining after the starch fraction of corn is fermented with selected yeasts to produce ethanol and carbon dioxide. After complete fermentation, the alcohol is removed by distillation and the remaining fermentation residues are dried.

In some advantageous embodiments, the by-product is selected from the group consisting of distillers' wet grain (DWG), distillers' dried grains (DDG), distillers' solubles (DS), distillers' dried solubles (DDS), distillers' dried grain with solubles (DDGS), and mixtures thereof. In particular, the by-product having mycotoxin binding abilities is DDGS.

Stillage is the product, which remains after the mash has been converted to sugar, fermented and distilled into ethanol. Stillage can be separated into two fractions, such as, by centrifugation or screening: (1) wet cake (solid phase) and (2) the thin stillage (supernatant). The solid fraction or distillers' wet grain (DWG) can be pressed to remove excess moisture and then dried to produce distillers' dried grains (DDG). After ethanol has been removed from the liquid fraction, the remaining liquid can be evaporated to concentrate the soluble material into condensed distillers' solubles (DS) or dried and ground to create distillers' dried solubles (DDS). DDS is often mixed with DDG to form distillers' dried grain with solubles (DDGS). DDG, DDGS, and DWG are collectively referred to as distillers' grain(s).

In one embodiment of the present disclosure enzymes were added during and/or preferably after the fermentation in the production process to the fermented mash and before the separation step like distillation, where the desired fermentation main product is separated from the rest of the fermented mash. The enzymes according to the present disclosure were capable of degrading components in the fermented mash (beer or beer mash) which improves the quality of by-products in respect to prebiotic content or residues like brewer's grains, dried distiller's grains, dried distiller's solubles, distiller's dried grains with solubles, WDG or/and residues of the cereal processing industry, or mixtures thereof. Components of the fermented mash can be cell walls, cell-walls of fermenting microorganisms like yeasts, microorganisms etc.

Surprisingly, the degradation of the fermenting microorganisms itself by adding the enzyme composition according to the present disclosure, in particular by using the beta 1,3 glucanase and/or the beta 1,6 glucanase, particularly in combination with a xylanase and/or a mannanase and /or protease , results in an increase of the prebiotic content in the beer mash which results in an improvement of the prebiotic quality like the increase of beta glucans and / or mannan-oligo-saccharides (mos) content of the byproducts resulting from enzymatic hydrolysis of the fermentative organism cell wall like the yeast cell wall.

Therefore, in advantageous embodiments, the enzyme compositions used in the methods according to the present disclosure are capable to degrade the cell wall components of the fermenting organisms after the fermentation step.

In one aspect of the present disclosure, the prebiotic quality of by-products from a fermentative production process like DDG, DDGS,WDG or syrup can be improved with the methods according to the present disclosure by increasing the beta glucans and / or mannan-oligo-saccharides content and therefore the prebiotic quality of the by-products.

The method of the disclosure may be used on beer derived from production of any suitable fermentation product. The feedstock for producing the fermentation product may be any starch- and/or sugar containing material, preferably starch- and/or sugar containing plant material, including: sugar cane, tubers, roots, whole grain; and any combination thereof.

The starch-containing material may be obtained from cereals. Suitable starch-containing material includes corn (maize), wheat, barley, cassava, sorghum, rye, triticale, potato, or any combination thereof.

Corn is the preferred feedstock, especially when the fermentation product is ethanol. The starch-containing material may also consist of or comprise, e.g., a side stream from starch processing, e.g., C6 carbohydrate containing process streams that may not be suited for production of syrups. Beer components include fiber, hull, germ, oil and protein components from the starch-containing feedstock as well as non-fermented starch, yeasts, yeast cell walls and residuals. Production of a fermentation product is typically divided into the following main process stages: a) Reducing the particle size of starch-containing material, e.g., by dry or wet milling; b) Cooking the starch-containing material in aqueous slurry to gelatinize the starch, c) Liquefying the gelatinized starch-containing material in order to break down the starch (by hydrolysis) into maltodextrins (dextrins); d) Saccharifying the maltodextrins (dextrins) to produce low molecular sugars (e.g., DP1- 2) that can be metabolized by a fermenting organism; e) Fermenting the saccharified material using a suitable fermenting organism directly or indirectly converting low molecular sugars into the desired fermentation product; f) Recovering the fermentation product, e.g., by distillation in order to separate the fermentation product from the fermentation mash.

As also explained above beer (or fermented mash) is the fermentation product consisting of ethanol, other liquids and solids of a desired fermentation product. According to the disclosure the primary fermentation product may be any fermentation product, including alcohols (e.g., ethanol, methanol, butanol, 1,3-propanediol); organic acids (e.g., citric acid, acetic acid, itaconic acid, lactic acid, gluconic acid, gluconate, succinic acid, 2,5-diketo-D-gluconic acid); ketones (e.g., acetone); amino acids (e.g., glutamic acid); gases (e.g., $H_2$ and $CO_2$), and more complex compounds, including, for example, antibiotics (e.g., penicillin and tetracycline); enzymes; vitamins (e.g., riboflavin, B12, beta-carotene); and hormones. Fermentation is also commonly used in the production of consumable alcohol (e.g., spirits, beer and wine), dairy (e.g., in the production of yogurt and cheese), leather, and tobacco industries. In a preferred embodiment the fermentation product is a liquid, preferably an alcohol, especially ethanol. The beer contemplated according to the invention may be the product resulting from a fermentation product production process including above mentioned steps a) to f). However, the beer may also be the product resulting from other fermentation product production processes based on starch- and/or lignocellulose containing starting material.

The fermenting organism may be a fungal organism, such as yeast, or bacteria. Preferred organisms for ethanol production are yeasts, such as e.g. *Pichia* or *Saccharomyces*. Preferred yeasts according to the disclosure are *Saccharomyces* species, in particular *Saccharomyces cerevisiae* or baker's yeast.

In a further advantageous embodiment, the disclosure pertains to methods of producing a prebiotic animal feed product comprising a fermentation by-product having a high content of prebiotics like beta glucans and/or mannan-oligo-saccharides from starch-containing material in an alcohol fermentation production process, said method comprising the steps of:

vi) Converting the starch-containing material to fermentable sugars,

vii) Fermentation of the fermentable sugars with a fermenting microorganism to fermented mash,

viii) Separation of the alcohol in the fermented mash by distillation,

ix) Subjecting the remaining whole stillage to an enzyme composition capable of degrading one or more components of the whole stillage to beta glucans and/or mannan-oligo-saccharides,

x) Separation of the by-product.

After the enzymatic digestion in the whole stillage the by-product is separated and the wet cake and the thin stillage are separated.

Further, by adding the enzymes according to the present disclosure to the fermented mash before the distillation step is an advantage since the enzymes in the enzyme compositions are inactivated during the distillation.

Due to the improved quality, the by-products and residues can be used as high quality animal feed with an increaded content of the prebiotics beta glucan and mannan-oligo-saccharides (mos).

In one embodiment, the disclosure relates to methods for formulating prebiotically useful feed additives as co-products of the above-referenced methods for improving prebiotic characteristics of a food and feed product containing microorganisms.

The processes for producing fermentation products includes the production of a large number of fermentation products comprising but not limited to alcohols (in particular ethanol) ; acids, such as citric acid, itaconic acid, lactic acid, gluconic acid, lysine ; ketones; amino acids, such as glutamic acid, but also more complex compounds such as antibiotics, such as penicillin, tetracyclin ; enzymes; vitamins, such as riboflavin, B12, beta-carotene; hormones, such as insulin. Preferred is drinkable ethanol as well as industrial and fuel ethanol.

Processes for producing fermentation products, such as ethanol, from a starch or lignocellulose containing material are well known in the art. The preparation of the starch- containing material such as corn for utilization in such fermentation processes typically begins with grinding the corn in a dry-grind or wet-milling process. Wet-milling processes involve fractionating the corn into different components where only the starch fraction enters into the fermentation process. Dry-grind processes involve grinding the corn kernels into meal and mixing the meal with water and enzymes. Generally two different kinds of dry-grind processes are used. The most commonly used process, often referred to as a "conventional process," includes grinding the starch-containing material and then liquefying gelatinized starch at a high temperature using typically a bacterial alpha-amylase, followed by simultaneous saccharification and fermentation (SSF) carried out in the presence of a glucoamylase and a fermentation organism. Another well-known process, often referred to as a "raw starch hydrolysis" process (RSH process), includes grinding the starch-containing material and then simultaneously saccharifying and fermenting granular starch below the initial gelatinization temperature typically in the presence of an acid fungal alpha-amylase and a glucoamylase.

In a process for producing ethanol from corn, following SSF or the RSH process the ethanol is distilled from the whole mash after fermentation. The resulting ethanol-free slurry, usually referred to as whole stillage, is separated into solid and liquid fractions (i.e., wet cake and thin stillage containing about 35 and 7% solids, respectively). The thin stillage is often condensed by evaporation into a thick stillage or syrup and recombined with the wet cake and further dried into distillers' dried grains with solubles distillers' dried grain with solubles (DDGS) for use in animal feed.

The method may be used on beer derived from production of any suitable fermentation product. The feedstock for producing the fermentation product may be any starch- and/or sugar containing material, preferably starch- and/or sugar containing plant material, including: sugar cane, tubers, roots, whole grain; and any combination thereof.

The starch-containing material may be obtained from cereals. Suitable starch-containing material includes corn (maize), wheat, barley, cassava, sorghum, rye, triticale, potato, or any combination thereof.

As mentioned above, beer (or fermented mash) is the fermentation product consisting of ethanol, other liquids and solids of a desired fermentation product. According to the invention the fermentation product may be any fermentation product, including alcohols (e.g., ethanol, methanol, butanol, 1,3-propanediol); organic acids (e.g., citric acid, acetic acid, itaconic acid, lactic acid, gluconic acid, gluconate, succinic acid, 2,5-diketo-D-gluconic acid); ketones (e.g., acetone); amino acids (e.g., glutamic acid); gases (e.g., $H_2$ and $CO_2$), and more complex compounds, including, for example, antibiotics (e.g., penicillin and tetracycline); enzymes; vitamins (e.g., riboflavin, B12, beta-carotene); and hormones. Fermentation is also commonly used in the production of consumable alcohol (e.g., spirits, beer and wine), dairy (e.g., in the production of yogurt and cheese), leather, and tobacco industries. In a preferred embodiment the fermentation product is a liquid, preferably an alcohol, especially ethanol. The beer contemplated according to the invention may be the product resulting from a fermentation product production process. However, the beer may also be the product resulting from other fermentation product production processes based on starch- and/or lignocellulose containing starting material.

Enzymes used for degrading beer components include carbohydrases such as alpha-amylase, glucoamylase, cellulase and/or hemicellulases, such as mannanases, xylanases and beta-glucanases, pectinases and proteases, or a mixture thereof.

In advantageous embodiment, the enzyme compositions comprise a beta-glucanase, in particular for the degradation of the cell walls from the fermenting microorganisms for increasing the beta glucans and/or mannan-oligo-saccharides content. To avoid the degradation of the fermentative microorganisms the enzyme composition is added after the fermentation step. As used herein "after the fermentation" or "after the fermentation step" means that a large part, in particular at least 80%, preferably at least 90% or all of the fermentable sugars like glucose are converted to the desired fermentation products such as ethanol.

In an advantageous embodiment, the enzyme composition comprises a beta-glucanase, in particular a beta-1,3-glucanase. The cell walls of most true fungal and yeast microorganisms contain a network of glucan, which gives the cell wall strength. Further major fungal cell walls constituents are mannoprotein and chitin. For the degradation of the fermented mash components, in particular for the degradation of the fibers and the fermenting microorganisms the following enzymes may be used.

Beta-1,3-glucanases as used herein are enzymes capable of degrading of glucan. Glucan and chitin are far more resistant to microbial degradation than cellulose, which is the major constituent of the cell wall of many yeasts and fungi-like organisms. Glucan is predominantly beta -1,3-linked with some branching via 1,6-linkage (Manners et al., Biotechnol. Bioeng, 38, p. 977, 1973), and is known to be degradable by certain beta -1,3-glucanase systems. beta -1,3-glucanase includes the group of endo-beta -1,3-glucanases also called laminarinases (E.C. 3.2.1.39 and E.C. 3.2.1.6, Enzyme Nomenclature, Academic Press, Inc. 1992).

A number of beta -1,3-glucanase genes and uses thereof have been disclosed in the prior art. An example is DD 226012 (Akad. Wissenshaft, DDR) which concerns a method for production of a Bacillus beta -1,3-glucanase. Further, JP 61040792 A (DOI K) describes a cell wall-cytolase beta -1,3-glucanase recombinant plasmid for removing the cell walls of yeast. The gene is derived from Arthrobacter and is transformed in *Escherichia* group bacteria. EP 440.304 concerns plants provided with improved resistance against pathogenic fungi transformed with at least one gene encoding an intracellular chitinase, or in intra- or extracellular beta -1,3-glucanase. The matching recombinant polynucleotides is also disclosed. WO 87/01388 (The Trustees of Columbia University) describes a method for preparing cell lytic enzymes, such as beta -1,3-glucanases, which can be produced by Oerksovia. WO 92/03557 (Majesty (Her) in Right of Canada) discloses a recombinant DNA expression vector comprising a 2.7 kb DNA sequence, derived from Oerskovia xanthineolytica, encoding a beta -1,3-glucanase. From WO 92/16632 a recombinant DNA sequence coding for a novel protein with beta -1,3-glucanase activity, is known.

Examples for commercial available beta -1,3-glucanase are Rohalase BX from AB Enzymes and BluZyD from Direvo Indunstrial Biotechnology GmbH.

Hemicellulases as used herein are enzymes capable to break down hemicellulose. Any hemicellulase suitable for use in hydrolyzing hemicellulose, preferably into xylose, may be used. Preferred hemicellulases include acetylxylan esterases, endo-arabinases, exo-arabinases, arabinofuranosidases, feruloyl esterase, endo-galactanases, exo-galactanases, glucuronidases, mannases, xylanases, and mixtures of two or more thereof. Preferably, the hemicellulase for use in the present invention is an exo-acting hemicellulase, and more preferably, the hemicellulase is an exo-acting hemicellulase which has the ability to hydrolyze hemicellulose under acidic conditions of below pH 7, preferably pH 3-7.

In one aspect, the hemicellulase(s) comprises a commercial hemicellulolytic enzyme preparation. Examples of commercial hemicellulolytic enzyme preparations suitable for use in the present invention include, for example, SHEARZYME(TM) (Novozymes A/S), CELLIC(TM) HTec (Novozymes A/S), CELLIC(TM) HTec2 (Novozymes A/S), VISCOZYME(R) (Novozymes A/S), ULTRAFLO(R) (Novozymes A/S), PULPZYME(R) HC (Novozymes A/S), MULTIFECT(R) Xylanase (Genencor), ACCELLERASE(R) XY (Genencor), ACCELLERASE(R) XC (Genencor), ECOPULP(R) TX-200A (AB Enzymes), HSP 6000 Xylanase (DSM), DEPOL(TM) 333P (Biocatalysts Limit, Wales, UK), DEPOL(TM) 740L. (Biocatalysts Limit, Wales, UK), and DEPOL(TM) 762P (Biocatalysts Limit, Wales, UK).

Preferably, the hemicellulase for use in the present disclosure is an endo-acting hemicellulase, which has the ability to hydrolyze hemicellulose under acidic conditions of below pH 7. An example of hemicellulase suitable for use in the present invention includes VISCOZYME L(TM) (available from Novozymes A/S, Denmark), Rohament GMP ™ (available from AB Enzymes).

In an embodiment the hemicellulase is a xylanase. In an embodiment the xylanase may preferably be of microbial origin, such as of fungal origin (e.g., Aspergillus, Fusarium, Humicola, Meripilus, Trichoderma) or from a bacterium (e.g., Bacillus). In a preferred embodiment the xylanase is derived from a filamentous fungus, preferably derived from a strain of Aspergillus, such as Aspergillus aculeatus; or a strain of Humicola, preferably Humicola lanuginosa. Examples of xylanases useful in the methods of the present invention include, but are not limited to, Aspergillus aculeatus xylanase (GeneSeqP:AAR63790; WO 94/21785), Aspergillus fumigatus xylanases (WO 2006/078256), and Thielavia terrestris NRRL 8126 xylanases (WO 2009/079210). The xylanase may preferably be an endo-1 ,4-beta-xylanase, more preferably an endo-1,4-beta-xylanase of GH 10 or GH 1 1. Examples of commercial xylanases include SHEARZYME(TM), BIOFEED WHEAT(TM), HTec and HTec2 from Novozymes A/S, Denmark.

Examples of beta-xylosidases useful in the methods of the present invention include, but are not limited to, Trichoderma reesei beta-xylosidase (UniProtKB/TrEMBL accession number Q92458), Talaromyces emersonii (Swiss-Prot accession number Q8X212), and Neurospora crassa (SwissProt accession number Q7SOW4).

According to the invention beer may in step i) be subjected to an effective amount of any xylanase (EC 3.2.1.8), such as any of below mentioned xylanases. Xylanase activity may be derived from any suitable organism, including fungal and bacterial organisms. Fungal xylanases may be derived from strains of genera including Aspergillus, Disporotrichum,Penicillium, Neurospora, Fusarium and Trichoderma.

Examples of suitable bacterial xylanases include include xylanases derived from a strain of Bacillus, such as Bacillus subtilis, such as the one disclosed in US patent no. 5,306,633 or

Contemplated commercially available xylanases include SHEARZYM E (TM), BIOFEED WHEAT(TM), (from Novozymes AJS), Econase CE ™ (from AB Enzymes), Depol 676 ™ (from Biocatalysts Ltd.) and SPEZYME(TM) CP (from Genencor Int.).).

Xylanase may be added in an amount effective in the range from $0.16 \times 10^6$ - $460 \times 10^6$ Units per ton beer mash.

Mannanases are hemicellulases classified as EC 3.2.1.78, and called endo-1 ,4-beta- mannosidase. Mannanase includes beta-mannanase, endo-1,4-mannanase, and galacto- mannanase. Mannanase is preferably capable of catalyzing the hydrolysis of 1 ,4-beta-D- mannosidic linkages in mannans, including glucomannans, galactomannans and galactoglu- comannans. Mannans are polysaccharides primarily or entirely composed of D-mannose units. The mannanase may be of any origin such as a bacterium or a fungal organism. In a specific embodiment the mannanase is derived from a strain of the filamentous fungus genus Trichoderma, preferably Trichoderma reseei. In an embodiment the mannanase is one of the mannanases described in WO2008/009673.

Mannanases have been identified in several Bacillus organisms. For example, Talbot et al., Appl. Environ. Microbiol., Vol.56, No. 11, pp. 3505-3510 (1990) describes a beta- mannanase derived from Bacillus stearothermophilus. Mendoza et al., World J. Microbiol. Biotech., Vol. 10, No. 5, pp. 551-555 (1994) describes a beta-mannanase derived from Bacillus subtilis. JP-A-03047076 discloses a beta-mannanase derived from Bacillus sp. JP-A- 63056289 describes the production of an alkaline, thermo stable beta-mannanase. JP-A- 63036775 relates to the Bacillus microorganism FERM P-8856 which produces beta- mannanase and beta-mannosidase. JP-A-08051975 discloses alkaline beta-mannanases from alkalophilic Bacillus sp. AM-001. A purified mannanase from Bacillus amyloliquefaciens is disclosed in WO 97/11164. WO 91/18974 describes a hemicellulase such as a glucanase, xylanase or mannanase active. Examples of commercially available mannanases include GAMANASE (TM) available from Novozymes A/S Denmark and Rohapect GMP ™ available from AB Enzymes GmbH.

Mannanase may be added in an amount effective in the range from $0.3 \times 10^6$ - $1.6 \times 10^6$ Units per ton beer mash.

A cellulase, used in accordance with the disclosure, may be any cellulase, in particular of microbial origin, in particular fungal or bacterial origin such as a cellulase derivable from a strain of a filamentous fungus (e.g., Aspergillus, Trichoderma, Humicola, Fusarium). Preferably, the cellulase acts on both cellulosic and lignocellulosic material. Preferred cellulases for use in the present invention include endo-acting cellulases, exo-acting celluases and cellobiases, and combinations thereof.. Examples of commercially available cellulases suitable according to the present invention include, for example, CELLULCLAST(TM) (available from Novozymes A/S), , LAMINEX(TM) and SPEZYME(TM) CP (Genencor Int.) and Econase CE ™ (from AB Enzymes GmbH), Rohalase BX™ (from AB Enzymes GmbH), Cellulase 13P ™ (from Biocatalysts Ltd.). Cellulase may be added in amounts effective in the range or from $0.03 \times 10^{<6>}$ -$16 \times 10^{<6>}$ Units per ton substrate (in beer mash)

Proteases as used in the present disclosure are enzymes that catalyze the cleavage of peptide bonds. Suitable proteases include fungal and bacterial proteases. Preferred proteases are acidic proteases, i.e., proteases characterized by the ability to hydrolyze proteins under acidic conditions below pH 7

Suitable acid fungal proteases include fungal proteases derived from Aspergillus, Mucor, Rhizopus, Candida, Coriolus, Endothia, Enthomophtra, Irpex, Penicillium, Sclerotium and Toru- lopsis. Commercial proteases include GC 106(TM) and SPEZYME(TM) FAN (available from Genencor, USA). Suitable bacterial proteases, although not acidic proteases, include the commercially available products ALCALASE(TM) and NEUTRASE(TM) (available from Novozymes A/S).

Protease may be added in an amount effective in the range from $0.002 \times 10^6$-$314 \times 10^6$ Units per ton beer mash.

In another aspect the present disclosure relates to method of producing a fermentation by-products having a high content of prebiotics like beta glucan and/or mannan-oligo-saccharides from starch containing material in a fermentation production process, said method comprising the steps of:

i) Converting the starch containing material to fermentable sugars,
ii) Fermentation of the fermentable sugars with a fermenting microorganism to fermented mash,
iii) Subjecting the fermented mash after the fermentation process to an enzyme composition capable of degrading one or more fermented mash components,
iv) Separation of the primary product in the fermented mash,
v) Separation of the fermentation by-product for using as an animal feed product.

In an advantageous embodiment, the fermentation production process is an ethanol production process. Therefore, the primary product is ethanol. In one embodiment, the ethanol as the primary product is separated by distillation.

Converting starch-containing material to fermentable sugars can be done by (a) liquefying a starch-containing material and (b) saccharifying the liquefied material obtained in step (a).

The liquefaction is preferably carried out in the presence of an alpha-amylase, preferably a bacterial alpha-amylase or acid fungal alpha-amylase. In an embodiment, a pullulanase, isoamylase, and and/or phytase is added during liquefaction.

Preferred organisms for ethanol production are yeasts, such as e. g. Pichia or Saccharomyces. Preferred yeast according to the disclosure is Saccharomyces species, in particular *Saccharomyces cerevisiae* or baker's yeast. The yeast cells may be added in amounts of $10^5$ to $10^{12}$, preferably from $10^7$ to $10^{10}$, especially $5 \times 10^7$ viable yeast count per ml of fermentation broth. During the ethanol producing phase the yeast cell count should preferably be in the range from $10^7$ to $10^{10}$, especially around $2 \times 10^8$. Further guidance in respect of using yeast for fermentation can be found in, e. g.,"The alcohol Textbook" (Editors K. Jacques, T. P. Lyons and D. R. Kelsall, Nottingham University Press, United Kingdom 1999).

The microorganism used for the fermentation is added to the mash and the fermentation is ongoing until the desired amount of fermentation product is produced; in a preferred embodiment wherein the fermentation product is ethanol to be recovered this may, e. g. be for 24-96 hours, such as 35-60 hours. The temperature and pH during fermentation is at a temperature and pH suitable for the microorganism in question and with regard to the intended use of the fermentation product, such as, e. g. , in an embodiment wherein the fermenting organism is yeast and the product is ethanol for recovery the preferred temperature is in the range about 26-34 C, e. g. about 32 C, and at a pH e. g. in the range about pH 3-6, e. g. about pH 4-5.

In another embodiment wherein the fermenting organism is yeast, and the fermented mash is to be used as a beer, the temperature of the mash the preferred temperature is around 10 to 35 °C.

As mentioned above, the fermenting organism is preferably yeast, e.g., a strain of *Saccharomyces cerevisiae* or *Saccharomyces diastaticus.* In an avantegous embodiment a yeast strain of *Saccharomyces diastaticus* is used (SIHA Amyloferm®, E. Begerow GmbH&Co, Langenlonsheim, Germany) since their exo-amylase activity can split liquid starch and also dextrin, maltose and melibiose.

In the liquefaction step the gelatinized starch (downstream mash) is broken down (hydrolyzed) into maltodextrins (dextrins). To achieve starch hydrolysis a suitable enzyme, preferably an alpha-amylase, is added. Liquefaction may be carried out as a three-step hot slurry process. The slurry is heated to between 60-95°C, preferably 80-85°C, and an alpha-amylase may be added to initiate liquefaction (thinning). Then the slurry may be jet-cooked at a temperature between 95-140°C, preferably 105-125°C, for about 1-15 minutes, preferably for about 3-10 minutes, especially around about 5 minutes. The slurry is cooled to 60-95°C and more alpha-amylase may be added to complete the hydrolysis (secondary liquefaction). The liquefaction process is usually carried out at a pH of 4.0 to 6.5, in particular at a pH of 4.5 to 6.

The saccharification step and the fermentation step may be performed as separate process steps or as a simultaneous saccharification and fermentation (SSF) step. The saccharification is carried out in the presence of a saccharifying enzyme, e. g. a glucoamylase, a beta-amylase or maltogenic amylase. Optionally a phytase and/or a protease is added.

Saccharification may be carried out using conditions well known in the art with a saccharifying enzyme, e.g., beta-amylase, glucoamylase or maltogenic amylase, and optionally a debranching enzyme, such as an isoamylase or a pullulanase. For instance, a full saccharification process may last up to from about 24 to about 72 hours, however, it is common to do a pre-saccharification for typically 40-90 minutes at a temperature between 30-65°C, typically about 60°C, followed by complete saccharification during fermentation in a simultaneous saccharification and fermentation process (SSF process). Saccharification is typically carried out at a temperature from 20-75°C, preferably from 40-70°C, typically around 60°C, and at a pH between 4 and 5, normally at about pH 4.5.

The most widely used process to produce a fermentation product, especially ethanol, is the simultaneous saccharification and fermentation (SSF) process, in which there is no holding stage for the saccharification, meaning that a fermenting organism, such as a yeast, and enzyme(s), including the hemicellulase(s) and/or specific endoglucanase(s), may be added together. SSF is typically carried out at a temperature from 25°C to 40°C, such as from 28°C to 35°C, from 30°C to 34°C, preferably around about 32°C. In an embodiment, fermentation is ongoing for 6 to 120 hours, in particular 24 to 96 hours.

After the fermentation, the fermented mash is subjected to an enzyme composition according to the present disclosure. In an embodiment, the enzyme composition comprises a beta-glucanase.

In a particular embodiment, the process further comprises, prior to liquefying the starch-containing material the steps of:

- reducing the particle size of the starch-containing material, preferably by milling; and
- forming a slurry comprising the starch-containing material and water.

The aqueous slurry may contain from 10-55 w/w % dry solids (DS), preferably 25-45 w/w % dry solids (DS), more preferably 30-40 w/w % dry solids (DS) of the starch-containing material. The slurry is heated to above the gelatinization temperature and an alpha-amylase, preferably a bacterial and/or acid fungal alpha-amylase, may be added to initiate liquefaction (thinning). The slurry may be jet-cooked to further gelatinize the slurry before being subjected to an alpha-amylase in step (a).

In a preferred embodiment, the starch containing material is milled cereals, preferably barley or corn, and the methods comprise a step of milling the cereals before step (a). In other words, the disclosure also encompasses methods, wherein the starch containing material is obtainable by a process comprising milling of cereals, preferably dry milling, e. g. by hammer or roller mils. Grinding is also understood as milling, as is any process suitable for opening the individual grains and exposing the endosperm for further processing. Two processes of milling are normally used in alcohol production: wet and dry milling. The term "dry milling" denotes milling of the whole grain. In dry milling the whole kernel is milled and used in the remaining part of the process Mash formation. The mash may be provided by forming a slurry comprising the milled starch containing material and brewing water. The brewing water may be heated to a suitable temperature prior to being combined with the milled starch containing material in order to achieve a mash temperature of 45 to 70°C, preferably of 53 to 66°C, more preferably of 55 to 60°C. The mash is typically formed in a tank known as the slurry tank.

Subsequent to fermentation the fermentation product may be separated from the fermentation medium. The slurry may be distilled to extract the desired fermentation product or the desired fermentation product from the fermentation medium by micro or membrane filtration techniques. Alternatively the fermentation product may be recovered by stripping. Methods for recovering fermentation products are well known in the art. Typically, the fermentation product, e.g., ethanol, with a purity of up to, e.g., about 96 vol. % ethanol is obtained.

Following the completion of the fermentation process, the material remaining is considered the whole stillage. As used herein, the term "whole stillage" includes the material that remains at the end of the fermentation process both before and after recovery of the fermentation product, e.g., ethanol. The fermentation product can optionally be recovered by any method known in the art. In one embodiment, the whole stillage is separated or partitioned into a solid and liquid phase by one or more methods for separating the thin stillage from the wet cake. Such methods include, for example, centrifugation and decanting. The fermentation product can be optionally recovered before or after the whole stillage is separated into a solid and liquid phase.

Thus, in one embodiment, the methods of the disclosure further comprise distillation to obtain the fermentation product, e.g., ethanol. The fermentation and the distillation may be carried out simultaneously and/or separately/sequentially; optionally followed by one or more process steps for further refinement of the fermentation product.

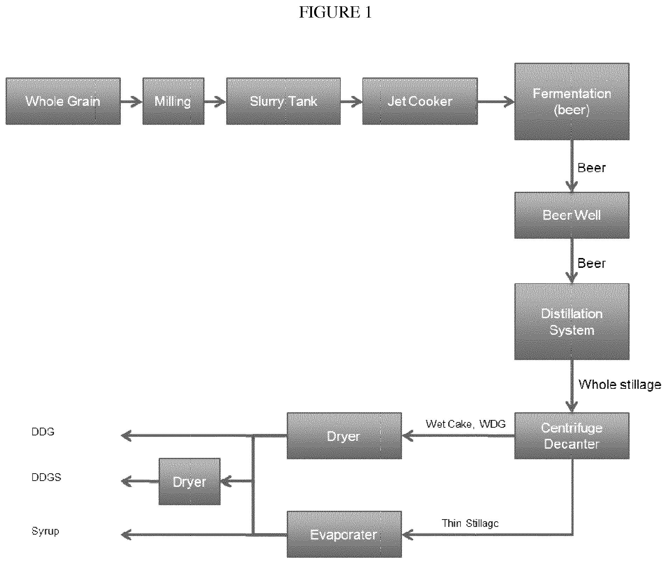

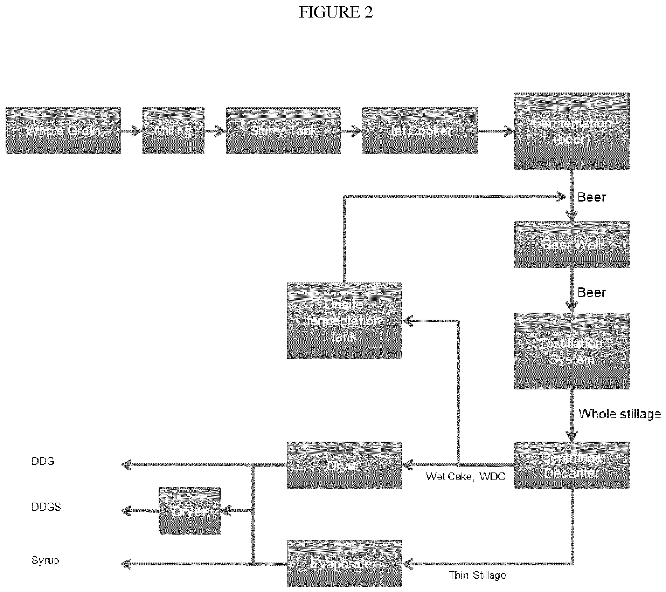

In an embodiment, the aqueous by-product (whole stillage) from the distillation process is separated into two fractions, e.g., by centrifugation: wet grain (solid phase), and thin stillage (supernatant). In another embodiment, the methods of the disclosure further comprise separation of the whole stillage produced by distillation into wet grain and thin stillage; and recycling thin stillage to the starch containing material prior to liquefaction. In one embodiment, the thin stillage is recycled to the milled whole grain slurry. The wet grain fraction may be dried, typically in a drum dryer. The dried product is referred to as distillers dried grains, and can be used as mentioned above as high quality animal feed. The thin stillage fraction may be evaporated providing two fractions (see Fig. 1 and Fig.2), (i) a condensate fraction of 4-6% DS (mainly of starch, proteins, and cell wall components), and (ii) a syrup fraction, mainly consisting of limit dextrins and non-fermentable sugars, which may be introduced into a dryer together with the wet grains (from the whole stillage separation step) to provide a product referred to as distillers dried grain with solubles, which also can be used as animal feed. Thin stillage is the term used for the supernatant of the centrifugation of the whole stillage. Typically, the thin stillage contains 4-6% DS (mainly starch and proteins) and has a temperature of about 60-90°C. In another embodiment, the thin stillage is not recycled, but the condensate stream of evaporated thin stillage is recycled to the slurry containing the milled whole grain to be jet cooked.

Further details on how to carry out liquefaction, saccharification, fermentation, distillation, and recovering of ethanol are well known to the skilled person.

Methods for dewatering stillage and for extracting oil from a fermentation product are known in the art. These methods include decanting or otherwise separating the whole stillage into wet cake and thin stillage. See, e.g., U.S. Patent Nos. 6,433,146, 7,601,858, and 7,608,729, and U.S. Application Publication No. 2010/0058649. Furthermore, the thin stillage can be evaporated or condensed into syrup or thick stillage from which the oil can be extracted utilizing centrifugation, filtering, heat, high temperature, increased pressure, or a combination of the same. Another way to extract oil is to lower the pH of the thin stillage or syrup. The use of surfactants to break emulsions also enhances oil extraction. Presses can also be used for dewatering. In one embodiment of the disclosure, the presence of beta 1,3-glucanase and/or xylanase in the fermented mash after the fermentation increases the amount of oil in the thin stillage and further the syrup or thick stillage.

The fermentation product(s) can be optionally recovered from the fermentation medium using any method known in the art including, but not limited to, chromatography, electrophoretic procedures, differential solubility, distillation, or extraction. For example, alcohol is separated from the fermented cellulosic material and purified by conventional methods of distillation as mentioned above. Ethanol with a purity of up to about 96 vol.% can be obtained, which can be used as, for example, fuel ethanol, drinking ethanol, i.e., potable neutral spirits, or industrial ethanol.

The inventions described and claimed herein are not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

EXAMPLES

**1. Material and Methods**

**1.1 Processes for producing fermentation products (e.g. mash)**

In one embodiment, the process of the production of ethanol form corn was performed as follows:

a) reducing the particle size of the starch-containing material by milling:

- corn was milled to < 2 mm particle size

b) forming a slurry comprising the starch-containing material and water

- 10 kg corn were tap water at 35°C to obtain a ~31,25% solid solution, total volume 30 liter

- pH range was 5.6 - 6.0

c) liquefying of the starch-containing material

- temperature was increased to 90°C

- 7 ml alpha-amylase (Novozymes Liquozyme SC) were added

- 1‰ antifoam was added (30 ml)

- incubation for 90 min at 90°C and 150 rpm

- slurry was cooled to 30°C, pH adjusted to ~4 with 1 $NH_2SO_4$

d) saccharifying of the liquefied material obtained

- 12 ml Glucoamylase (Novozymes Spirizyme Ultra) was added

e) fermentation

- Yeast propagation: 200 ml YNB-starch medium was incubated overnight (30°C, 150 rpm) and inoculated with 2 g yeast (SIHA Amyloferm), the complete pre-culture was added to the fermentation

- addition of 300 ppm ((NH4)2SO4) (10 g) as nitrogen source

- yeast addition

- pH was titrated to 4.0 with ammoniac solution (25%), supplies further nitrogen

- incubation for max. 48 h at 30°C and 100 rpm

- 2-ml samples were taken every 12 hrs to monitor fermentation progress (sugar-, ethanol concentration)

**1.2 Enzymes**

The following enzymes listed in Table 1 were used alone or in different combinations.Enzyme product activity determination is presented in C).

Table 1

| Typ | Activity | *Shortname* |
|---|---|---|
| beta 1-3 Glucanase | 10017 U/g | *Glu1* |

(continued)

| Typ | Activity | Shortname |
|---|---|---|
| beta 1-3 Glucanase | 763 U/ml | Glu5 |
| beta 1-3 Glucanase | 2588 U/ml | Glu9 |
| beta 1-3 Glucanase Xylanase | 1469 U/g 1835 U/g | Tre6 |
| Xylanase | 17047 U/g | BluZyD3 |

**1.3 Enzyme product activity determination:**

Substrates: Glucanase: beta-glucan from barley, low viscosity (Megazyme)
Xylanase: Xylan from Birchwood (Sigma)

Substrates were dissolved in buffer to a concentration of 0,8% (w/v)

Buffer: 50 mM NaAcetat, pH 4,5

The Enzymes were diluted in buffer, the right enzyme concentration must be determined for each enzyme. A $90 \mu l$ substrate and 10 $\mu l$ enzyme solution were mixed. A blank was measured replacing enzyme solution with water. Incubation for 30 min at 37°C (followed from 10 min at 4°C). Reducing sugars were measured after mixing of 50 $\mu l$ of the incubated substrate - enzyme mix with 50 $\mu l$ DNSA-Reagent.

The activity is calculated as Units per $\mu l$ or mg of enzyme product. 1 unit is defined as the amount of formed reducing ends in $\mu mol$ per minute. The enzyme activities are shown in Table 1.

**1.4 Analysis of beta-glucans**

Analysis of beta-glucans was carried out with the Yeast beta Glucan Kit K-EBHLG from Megazyme, but one fourth of the described volume in the instructions was used. The analysis was based on the specific enzymatic digestion of (1-3)(1-6)-beta-glucans to glucose. Controls carried out without enzymatic digestion of glucans provide the background concentrations of glucose, which have to be subtracted from the samples.

**1.5 Enzymatic digestion of beta-glucans**

$200 \mu L$ 1.2 M Na acetate was added to a $50 \mu L$ sample, which had been pipetted into an Eppendorf tube. Samples were treated with $5 \mu L$ Glucazyme™ an enzyme mixture containing beta-glucanase, beta-glucosidase and chitinase, then stirred by a plastic spatula and subsequently kept for 16 h at 40°C in an incubator. Glucazyme™ was left out in controls and $5 \mu L$ of distilled water was added instead.

**1.6 Analysis of glucose monomers derived from beta-glucans**

$25 \mu L$ both from the digestion samples and controls was added to $1000 \mu L$ GOPOD reagent (containing buffer adjusted to pH 7.4, glucose oxidase, peroxidase and 4-aminoantipyrine p-hydroxybenzoic acid) previously adjusted to 40°C in a water bath in a photometer cuvette (Rotilabo®-disposable cuvettes, polystirol). The cuvettes were incubated at 40°C for 30 minutes and the optical density at 510 nm was determined against air in the light path.

**1.7 Calculation of glucose**

Calculation of D-glucose concentration of samples and controls was carried with a glucose standard solution (1.5 g glucose/L). The absorbance of the water blank was subtracted from the absorbance of the sample, thereby obtaining $\Delta A_{D\text{-glucose\_sample}}$. This was also performed with the glucose standard $\Delta A_{D\text{-glucose\_standard}}$. Calculation of the glucose concentration of samples and controls was carried out by dividing $\Delta A_{D\text{-glucose}}$ by $\Delta A_{D\text{-glucose\_standard}}$ *1.5 g/L * dilution factor.

### 1.8 Calculation of beta-glucans

Glucose concentrations of the controls were subtracted from samples treated with Glucazyme™ providing the concentrations of beta-glucans in g/L glucose.

*Calculation of ⯑alculation of concentrations of beta-glucans in g/L glucose.amples treated wi*

The calculated beta-glucan concentrations (in glucose /L) of the untreated yeast cell and mash samples (i.e. controls) were set to 100%. From the data of beta-glucans in g/L glucose the relative concentrations with reference to the control was calculated.

### 1.9 Analysis of manno-oligosaccharides

Manno-oligosaccharides were analyzed by a varied method presented by Dallies N. *et al.* 1998 (Dallies N, François J, Paquet V. 1998. François J, Paquet V. A new method for quantitative determination of polysaccharides in the yeast cell wall. Application to the cell wall defective mutants of Saccharomyces cerevisiae. Yeast. 14(14):1297-1306), in which mannose concentrations were analyzed in samples, which had been treated after hot acid hydrolysis and without treatment (controls). The difference amounts of the mannose concentration of samples treated by hot acid hydrolysis and controls give the concentrations of manno-oligosaccharides (in concentration of the mannose oligomer) in the samples.

### 1.10 Hydrolysis of manno-oligosaccharides

500 $\mu$l 2M HCl was added to a 50 $\mu$L sample, which has been pipetted into a 1.5 ml Eppendorf tube. For hot acid hydrolysis the sample was kept at 95°C on an Eppendorf thermomixer shaken at 300 rpm for 2 hours. After heating the tube was cooled in an ice-bath for 5 minutes and 500 $\mu$l 2 M NaOH was added into the tube. 50 $\mu$l of the sample was used for enzymatic glucose / fructose / mannose analysis. Because determination of mannose was affected by high concentrations of glucose and fructose these sugars were also determined.

*Enzymatic analysis of glucose, fructose and mannose*

Glucose, fructose and mannose were analyzed in a disposable semi-micro (1.5 mL) photometer cuvette (Rotilabo®-disposable cuvettes, polystirol) at 340 nm at 25°C against air in the light path of the photometer Cary 50 (Varian) according to the D-mannose, D-fructose and D-glucose assay procedure of the analysis kit K-MANGL by the company Megazyme, but using half of the final volume as stated in the instructions: 1.26 mL instead of 2.52 mL for glucose analysis, 1.27 mL instead of 2.54 mL for fructose and 1.28 mL instead of 2.56 mL for mannose.

1 mL of distilled water was added to 50 $\mu$L of the sample. Then 100$\mu$l of bottle 1 of kit K-MANGL containing triethanolamine buffer pH 9 with magnesium chloride was added followed by 100$\mu$L of bottle 2 containing ATP and NADP. After each step the reaction mixture was stirred by plastic spatula. After 15 minutes incubation at 25°C optical density was determined at 340 nm against air in the light path giving the background (blank) extinction of the sample (A1). For analysis of glucose 10$\mu$L of a suspension of hexokinase and glucose-6-phosphate dehydrogenase from bottle 3 was pipetted, stirred by a plastic spatula and incubated for 45 Minutes at 25°C. Then the optical density at 340 nm (A2) was determined like A1. For analysis of fructose 10$\mu$L of a suspension of phosphoglucose isomerase from bottle 4 was added, stirred by a plastic spatula and incubated for 30 minutes at 25°C. Then the optical density at 340 nm (A3) was determined like A1. For analysis of mannose 10$\mu$L of a suspension of phosphomannose isomerase from bottle 5 was added, stirred by a plastic spatula and incubated for 45 minutes at 25°C. Then the optical density at 340 nm (A4) was determined like A1.

### 1.11 Calculation of glucose, fructose and mannose

The absorbance differences (A2-A1) for both blank and sample were determined. The absorbance difference of the blank was subtracted from the absorbance difference of the sample, thereby obtaining $\Delta A_{D\text{-glucose}}$.

The absorbance differences (A3-A2) for both blank and sample were determined. The absorbance difference of the blank was subtracted from the absorbance difference of the sample, thereby obtaining $\Delta A_{D\text{-fructose}}$.

The absorbance differences (A4-A3) for both blank and sample were determined. The absorbance difference of the blank was subtracted from the absorbance difference of the sample, thereby obtaining $\Delta A_{D\text{-mannose}}$.

The concentration of D-glucose, D-fructose and D-mannose can be calculated as follows:

$$c = \frac{V*MW}{\varepsilon*d*v} * \quad A \text{ [g/L]}$$

where:

V = final volume [mL]; MW = molecular weight of D-glucose, D-fructose or D-mannose [g/mol]
$\varepsilon$ = extinction coefficient of NADPH at 340 nm = 6300 [l * mol$^{-1}$ * cm-1]; d = light path [cm]
v = sample volume [mL]

*It follows for D-glucose:*

c = (1.26 * 180.16 * $\Delta A_{\text{D-glucose}}$) / (6300 * 1 * 0.05) [g/L] = 0.7206 * $\Delta A_{\text{D-glucose}}$ [g/L]

*for D-fructose:*

c = (1.27 * 180.16 * $\Delta A_{\text{D-fructose}}$) / (6300 * 1 * 0.05) [g/L] = 0.7264 * $\Delta A_{\text{D-fructose}}$ [g/L]

*for D-mannose:*

c = (1.28 * 180.16 * $\Delta A_{\text{D-mannose}}$) / (6300 * 1 * 0.05) [g/L] = 0.7321 * $\Delta A_{\text{D-mannose}}$ [g/L]

If the sample has been diluted during preparation, the result must be multiplied by the dilution factor, F.

*Calculation of manno-oligosaccharides*

The concentrations of mannose of samples (controls), which were not treated by hot acid hydrolysis, were subtracted from the samples treated by hot acid hydrolysis. The results give the concentrations of manno-oligosaccharides in mannose g/L.

**2. Example 1**

**Improvement of beta-glucan in DDGS extracts derived from enzyme treated mash**

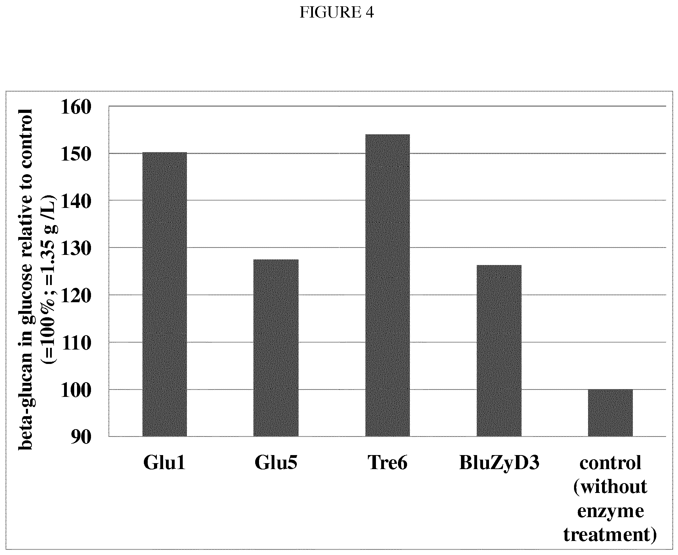

For analysis of the effects of enzyme treatment on mash, from which DDGS extracts were generated, 250 ml Erlenmeyer flasks were used as reaction batches, in which 200 ml of fermentatively produced mash were filled in. Enzyme stock solutions (100 mg/mL) solved in 50 mM Na-acetate, pH 4,5 buffer were prepared and 800 $\mu$L of the stock solution was added to the Erlenmeyer flask and incubated at 37°C for 18h at 150 rpm. Then the mash samples were dried (48h at 60°C) to produce DDGS. For the analysis of soluble beta-glucans, 1 g DDGS was dissolved in 3 ml water for 20 min at 50°C. The residual solids were removed by centrifugation at 4000 rpm for 15 minutes at 4°C and then the beta-glucan concentrations of the supernatants were analyzed according to (d).
By adding Glu1, Glu9, Tre6 or BluZyD3 to the mash beta-glucan concentration, i.e. beta-glucan content, was increased compared to the control (sample without enzyme treatment) as presented in table 2 and figure 4.

**Table 2**

|  | beta-glucan in g glucose / L | relative to control [100%] |
| --- | --- | --- |
| Glu1 | 2.02 | 150 |
| Glu9 | 1.72 | 127 |
| Tre6 | 2.08 | 153 |
| BluZyD3 | 1.70 | 126 |
| control (without enzyme treatment) | 1.35 | 100 |

Figure 4 shows the release of beta-glucans (in glucose) in DDGS extracts derived from enzymatic hydrolysis of mash treated by different enzymes. By adding Glu1, Glu9, Tre6 or BluZyD3 to the mash beta-glucan concentrations were increased in DDGS extracts compared to the control (without enzyme treatment).

## 3. Example 2

### Improvement of manno-oligosaccharides in DDGS extracts derived from enzyme treated mash

For analysis of the effects of enzyme treatment on mash, from which DDGS extracts were generated, 250 ml Erlenmeyer flasks were used as reaction batches, in which 200 ml of fermentatively produced mash were filled in. Enzyme stock solutions (100 mg/mL) solved in 50 mM Na-acetate, pH 4,5 buffer were prepared and 800 $\mu$L of the stock solution was added to the Erlenmeyer flask and incubated at 37°C for 18h at 150 rpm. Then the mash samples were dried (48h at 60°C) to produce DDGS. For the analysis of soluble manno-oligosaccharides, 1 g DDGS was dissolved in 3 ml water for 20 min at 50°C. The residual solids were removed by centrifugation at 4000 rpm for 15 minutes at 4°C and then the manno-oligosaccharides concentrations of the supernatants were analyzed according to (e).

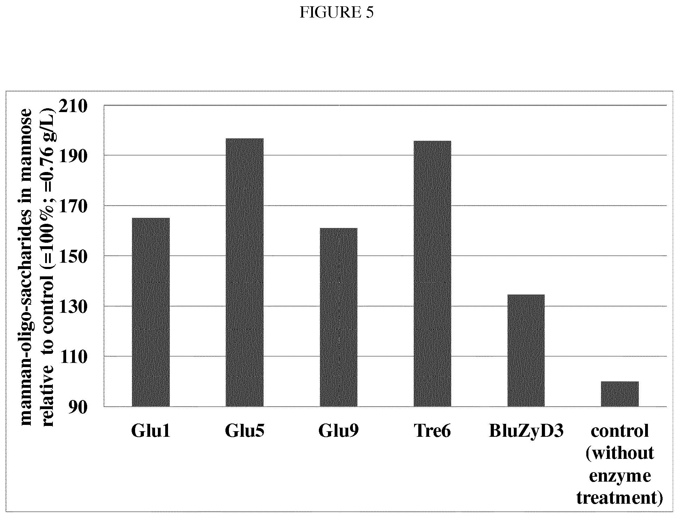

By adding Glu1, Glu5, Glu9, Tre6 or BluZyD3 to the mash manno-oligosaccharide (mos) concentration, i.e. manno-oligosaccharide (mos) content, was increased to the control (sample without enzyme treatment) as presented in table 3 and figure 5.

### Table 3

|  | mosing mannose /L | relative to control [100%] |
|---|---|---|
| Glu1 | 1.26 | 165 |
| Glu5 | 1,50 | 197 |
| Glu9 | 1.22 | 161 |
| Tre6 | 1.49 | 196 |
| BluZyD3 | 1.02 | 135 |
| control (without enzyme treatment) | 0.76 | 100 |

Figure 5 shows the release of mannan-oligo-saccharides (in mannose) in DDGS extracts derived from enzymatic hydrolysis of mash treated by different enzymes. By adding Glu1, Glu5, Glu9, Tre6 or BluZyD3 to the mash manno-oligosaccharide (mos) concentrations were increased in DDGS extracts compared to the control (without enzyme treatment).

## Claims

1. Use of an enzyme composition comprising a beta-1,3-glucanase and/or a xylanase for the improvement of the prebiotic quality of a by-product derived from fermented mash in a fermentative ethanol production process comprising the steps of: i) subjecting the fermented mash after the fermentation to said enzyme composition capable of degrading one or more fermented mash components, ii) separating the desired primary fermentation product, iii) separating the fermentation by-product having improved prebiotic quality, wherein the improvement of the prebiotic quality is an increase in the amount of manno-oligosaccharides

2. The use according to claim 1, wherein the by-product is selected from the group consisting of distillers' wet grain (DWG), distillers' dried grains (DDG), distillers' solubles (DS), distillers' dried solubles (DDS), distillers' dried grain with solubles (DDGS), and mixtures thereof.

3. The use according to claim 1, wherein the by-product is DDGS.

## Patentansprüche

1. Verwendung einer Enzymzusammensetzung, die eine Beta-1,3-Glucanase und / oder eine Xylanase umfasst, zur Verbesserung der präbiotischen Qualität eines Nebenprodukts, das aus fermentierter Maische stammt, in einem fermentativen Ethanol-Herstellungsverfahren, umfassend die Schritte: i) Aussetzen der fermentierten Maische nach der Fermentation mit der Enzymzusammensetzung, die eine oder mehrere fermentierte Maischekomponenten abbauen kann, ii) Trennen des gewünschten primären Fermentationsprodukts, iii) Trennen des Fermentationsnebenprodukts mit verbesserter präbiotischer Qualität, wobei die Verbesserung der präbiotischen Qualität eine Erhöhung der Menge an Manno-Oligosacchariden ist

2. Verwendung nach Anspruch 1, pe wobei das Nebenprodukt ausgewählt ist aus der Gruppe bestehend aus Distillers' Wet Grain (DWG), Distillers' Dried Grains (DDG), Distillers' Solubles (DS), Distillers' Dried Solubles (DDS), Distillers' Dried Grain with Solubles (DDGS), und Mischungen davon

3. Verwendung nach Anspruch 1, wobei das Nebenprodukt DDGS ist

**Revendications**

1. Utilisation d'une composition enzymatique comprenant une béta-1,3-glucanase et / ou une xylanase pour l'amélioration de la qualité prébiotique d'un sous-produit dérivé d'un moût fermenté dans un procédé de production d'éthanol fermentaire, comprenant les étapes de: i) soumission du moût fermenté après le processus de fermentation à ladite composition enzymatique capable de dégrader un ou plusieurs composants de moût fermenté, ii) séparer le produit de fermentation primaire désiré, iii) séparer le sous-produit de fermentation ayant une qualité prébiotique améliorée, où l'amélioration de la qualité prébiotique est une augmentation de la quantité de manno-oligosaccharides.

2. Utilisation selon la revendication 27, dans laquelle le sous-produit est choisi dans le groupe constitué des grains humides de distillerie (DWG), des grains séchés de distillerie (DDG), des solubles de distillerie (DS), des solubles séchés de distillerie (DDS), céréales séchées de distillerie avec solubles (DDGS) et leurs mélanges.

3. Utilisation selon la revendication 1, dans laquelle le sous-produit est le DDGS.

FIGURE 1

FIGURE 2

FIGURE 3

FIGURE 4

FIGURE 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6210686 B, Bell
- WO 9504467 A, Hayen and Pollmann
- US 5223491 A, Donzis
- US 5189028 A, Nikl and Allbright
- US 6306453 B1, Kürzinger
- US 5512287 A
- US 2009221806 A1
- DD 226012
- JP 61040792 A
- EP 440304 A
- WO 8701388 A
- WO 9203557 A
- WO 9216632 A
- WO 9421785 A
- WO 2006078256 A
- WO 2009079210 A
- US 5306633 A
- WO 2008009673 A
- JP 03047076 A
- JP 63056289 A
- JP 63036775 A
- JP 08051975 A
- WO 9711164 A
- WO 9118974 A
- US 6433146 B
- US 7601858 B
- US 7608729 B
- US 20100058649

**Non-patent literature cited in the description**

- **PRAPULLA et al.** *Adv Appl microbial,* 2000, vol. 47, 299-343
- **VAN LOO.** *Food Science and Technology Bulletin: Functional Foods,* 2005, vol. 2, 83-100
- **VAN LAERE et al.** *J Agric Food Chem,* 2000, vol. 48, 1644-1652
- **LEE et al.** *Anaerobe,* 2002, vol. 8, 319-324
- **HU et al.** *Anaerobe,* 2006, vol. 12, 260-266
- **WANG et al.** *Nutrition Research,* 2006, vol. 26, 597-603
- **VAZQUEZ et al.** *Industrial Crops and Products,* 2006, vol. 24, 152-159
- **PETRAVIC-TOMINAC et al.** *Conspectus Scientificus,* 2010, vol. 75 (4), 149-158
- **BROWN ; GORDON.** *Cell Microbiol,* 2005, vol. 7, 471-479
- **VOLMAN et al.** *Physiology and Behavior,* 2008, vol. 94, 276-284
- **BOHN ; BEMILLER.** *Carbohyd Polym,* 1995, vol. 28, 3-14
- **GARDINER.** *Glycoscience and Nutrition,* 2000, vol. 1, 1-6
- **VETVICKA et al.** *JANA,* 2002, vol. 5, 1-6
- **VETVICKA et al.** *Int J Biol Macromol,* 2007, vol. 40, 291-298
- **RICE et al.** *J Leukocyte Biol,* 2002, vol. 72, 140-146
- **DILUZIO.** *Trends Pharmacol Sci,* 1983, vol. 4, 344-347
- **KOUGIAS et al.** *Infect Immun,* 2001, vol. 69, 3933-3938
- **HONG et al.** *Cancer Res,* 2003, vol. 63, 9023-9031
- **OLIVEIRA et al.** *Toxicol In Vitro,* 2006, vol. 21, 41-52
- **KERNODL et al.** *Antimicrob Agents Chemother,* 1998, vol. 42, 545-549
- **DELLINGER et al.** *Arch Surg,* 1999, vol. 134, 977-983
- **WAKSHULL et al.** *Immunopharmacology,* 1999, vol. 41, 89-107
- **TZIANABOS ; CISNEROS.** *Ann N.Y. Acad Sci,* 1996, vol. 797, 285-287
- **KERNODLE et al.** *Antimicrob Agents Chemother,* 1998, vol. 42, 545-549
- **LEBLANC et al.** *J Leukoc Biol,* 2006, vol. 79, 667-675
- **ONDERDONK et al.** *Infect Immun,* 1992, vol. 60, 1642-1647
- **DILUZIO et al.** *Int J Cancer,* 1979, vol. 24, 773-779
- **KULICKE et al.** *Carbohyd Res,* 1997, vol. 297, 135-139
- **LIANG et al.** *Int J Immunopharmacol,* 1998, vol. 20, 595-614
- **TZIANABOS et al.** *J Infect Dis,* 1998, vol. 178, 200-206
- **CHEN ; SEVIOUR.** *Mycol Res,* 2007, vol. 111, 635-652

- What is beta-glucan?. **MASON.** Safe goods. New Century Publishing, 2000, 4-36
- **NICOLOSI et al.** *Am J Clin Nutr,* 1999, vol. 70, 208-212
- **KEOGH et al.** *Am J Clin Nutr,* 2003, vol. 78, 711-718
- **STERITI.** *Dynamic Chiropractis,* 2007, vol. 7, 1-6
- **NAUMANN et al.** *Amer J Clin Nutrit,* 2006, vol. 83, 601-605
- **BROWDER et al.** *Ann Surg,* 1990, vol. 211, 605-613
- **PORTERA et al.** *J Leukocyte Biol,* 1997, vol. 72, 140-146
- **VETVICKA.** *JANA,* 2001, vol. 3, 31-34
- **KELLER.** *International Journal of Pharmaceutical Compounding,* 2000, vol. 4, 342-345
- **COOK et al.** *Fish Shellfish Immun,* 2003, vol. 14, 333-345
- **LEE et al.** *Biomaterials,* 2003, vol. 24, 2503-2511
- **STRICKLING et al.** *An Feed Sci & Tech,* 2000, vol. 86, 205-219
- **SPRING et al.** *Poult Sci,* 2000, vol. 79, 205-211
- **HOOGE.** *Feedstuffs,* 2003, vol. 75 (1
- **OSBORN ; KHAN.** Oligosaccharides: Their synthesis and biological roles. Oxford University Press Inc, 2000
- **OFEK ; BEACHEY.** *Infection and Immunity,* 1978, vol. 22 (1), 247-253
- **OYOFO et al.** *Poult Sci,* 1989, vol. 68, 1351-1356
- **RÖCKENDORF et al.** *Aust J Chem,* 2002, vol. 55, 87-93
- **FERKET et al.** Benefits of Dietary Antibiotic and Mannanoligosaccharide Supplementation for Poultry. *Proc Multi-State Poultry Meeting,* 2002
- **IJI et al.** *J Sci Food Agric,* 2001, vol. 81, 1186-1192
- **DROLESKEY et al.** *Avian Diseases,* 1994, vol. 38, 275-281
- **OYOFO et al.** *Avian Diseases,* 1989, vol. 33, 531-534
- **FERNANDEZ et al.** *Avian Pathology,* 2002, vol. 31 (1), 49-58
- **FINUANCE et al.** *Poult Sci,* 1999, vol. 78 (1), 77
- **SWANSON et al.** *J Nutr,* 2002, vol. 132, 980-989
- **ZENTEK et al.** *J Nutr,* 2002, vol. 132, 1682S-1684S
- **SPEARMAN.** Effect on Mannan oligosaccharide (mos) supplementation on the immune status of mares and their foals. *Master thesis at the university of Florida,* 2004
- **FRITTS ; WALDROUP.** *Poult Sci,* 2000, vol. 79 (1), 126
- **HULET et al.** *Poult Sci,* 2000, vol. 79 (1), S186
- **STANLEY et al.** *Poult Sci,* 2000, vol. 79 (1), S186
- **BURESH et al.** *Poult Sci,* 1985, vol. 64, 1041-1042
- **HARMS et al.** *Poult Sci,* 1986, vol. 65, 1984-1986
- **O'QUINN et al.** *J Anim Sci,* 2001, vol. 79, 212
- **KIM et al.** *J Anim Sci Tech,* 2000, vol. 42 (4), 489-498
- Nutrient Requirements of Swine. National Academy Press, 1998
- **GILLEY et al.** *Trans Am Soc Agric Eng,* 2000, vol. 43, 1853-1859
- **LEMIEUX et al.** *J Anim Sci,* 2003, vol. 81, 2482-2487
- **DAVIS et al.** *J Anim Sci,* 2002, vol. 80, 2887-2894
- **HEINRICHS et al.** *J Dairy Sci,* 2003, vol. 86 (12), 4064-4069
- **BALLOU.** *J Biol Chem,* 1970, vol. 245 (5), 1197-1203
- **YOUNG.** *Glycoc J,* 1998, vol. 15, 815-822
- **SAVAGE et al.** *Poult Sci,* 1996, vol. 75 (1
- **MALZONE et al.** *Poult Sci,* 2000, vol. 79 (1), 165
- **SHASHIDHARA ; DEVEGOWDA.** *Poult Sci,* 2003, vol. 82 (8), 1319-1325
- **NEWMAN ; NEWMAN.** *J Anim Sci,* 2001, vol. 79, 189
- **MUCHMORE et al.** *J Leuko Biol,* 1990, vol. 48, 457-464
- **PODZORSKI et al.** *J Immunol,* 1990, vol. 144, 707-716
- **SPURLOCK.** *J Anim Sci,* 1997, vol. 75, 1773-1783
- **FRANKLIN et al.** *J Anim Sci,* 2002, vol. 80 (192
- **KUDOH et al.** *J Nutr Sci Vitaminol,* 1999, vol. 45 (2), 173-181
- **GUIGOZ et al.** *Nutr Res,* 2002, vol. 22, 13-25
- **CUNNINGHAM-RUNDLES ; LIN.** *Nutrition,* 1998, vol. 14, 573-579
- **MANNERS et al.** *Biotechnol. Bioeng,* 1973, vol. 38, 977
- Enzyme Nomenclature. Academic Press, Inc, 1992
- **TALBOT et al.** *Appl. Environ. Microbiol.,* 1990, vol. 56 (11), 3505-3510
- **MENDOZA et al.** *World J. Microbiol. Biotech.,* 1994, vol. 10 (5), 551-555
- The alcohol Textbook. Nottingham University Press, 1999

- **DALLIES N ; FRANÇOIS J ; PAQUET V.** A new method for quantitative determination of polysaccharides in the yeast cell wall. Application to the cell wall defective mutants of Saccharomyces cerevisiae. *Yeast,* 1998, vol. 14 (14), 1297-1306